# EUROPEAN PATENT SPECIFICATION

(11) **EP 1 096 952 B1**
(45) Date of publication and mention of the grant of the patent: **21.05.2008**
(21) Application number: 99934091.2
(22) Date of filing: 15.07.1999
(51) Int. Cl.: A61K 39/085, C07H 5/06, C07H 11/00, C07H 13/12, C08B 37/00

(54) **POLYSACCHARIDE VACCINE FOR STAPHYLOCOCCAL INFECTIONS**
POLYSACCHARID-IMPFSTOFF GEGEN STAPHYLOKOKKEN INFEKTIONEN
VACCIN DE POLYSACCHARIDE CONTRE LES INFECTIONS A STAPHYLOCCOQUES

(30) Priority: 15.07.1998 US 93117 P
(43) Date of publication of application: 09.05.2001
(60) Divisional application: 08101785.7
(73) Proprietor: THE BRIGHAM AND WOMEN'S HOSPITAL, INC., Boston, MA 02115 (US)
(72) Inventor: PIER, Gerald, B., Brookline, MA 02146 (US); MCKENNEY, David, Boston, MA 02115 (US); WANG, Ying, Boston, MA 02115 (US)
(74) Representative: Elend, Almut Susanne
(86) International application number: PCT/US1999/016129
(87) International publication number: WO 2000/003745

(56) References cited:
- WO-A-90/03398
- FR-A- 2 640 628
- US-A- 4 902 616
- MACK D ET AL: "The intercellular adhesin involved in biofilm accumulation of Staphylococcus epidermidis is a linear beta-1,6-linked glucosaminoglycan: Purification and structural analysis" JOURNAL OF BACTERIOLOGY, vol. 178, no. 1, January 1996 (1996-01), pages 175-183, XP000867576
- WESSELS M R ET AL: "Isolation and characterization of type IV group B Streptococcus capsular polysaccharide." INFECTION AND IMMUNITY, (1989 APR) 57 (4) 1089-94. , XP002129163
- LEE J C ET AL: "Chemical characterization and immunogenicity of capsular polysaccharide isolated from mucoid Staphylococcus aureus." INFECTION AND IMMUNITY, (1987 SEP) 55 (9) 2191-7. , XP002129164
- KOJIMA Y ET AL: "Antibody to the capsular polysaccharide/adhesin protects rabbits against catheter-related bacteremia due to coagulase-negative staphylococci" J INFECT DIS, vol. 162, no. 2, August 1990 (1990-08), pages 435-41, XP000867597 cited in the application
- FATTOM A ET AL: "Effect of conjugation methodology, carrier protein, and adjuvants on the immune response to Staphylococcus aureus capsular polysaccharides" VACCINE,GB,BUTTERWORTH SCIENTIFIC. GUILDFORD, vol. 13, no. 14, 1 October 1995 (1995-10-01), pages 1288-1293, XP004057429 ISSN: 0264-410X
- MCKENNEY D ET AL: "Broadly protective vaccine for Staphylococcus aureus based on an in vivo-expressed antigen." SCIENCE, (1999 MAY 28) 284 (5419) 1523-7. , XP002129165
- MCKENNEY D ET AL: "The ica locus of Staphylococcus epidermidis encodes production of the capsular polysaccharide /adhesin." INFECTION AND IMMUNITY, (1998 OCT) 66 (10) 4711-20. , XP002129166
- MAIRA-LITRAN ET AL: 'Immunochemical properties of the staphylococcal poly-N-Acetylglucosamine surface polysaccharide' INFECTION AND IMMUNITY vol. 70, no. 8, 2002 - 2002, pages 4433 - 4440
- MOREAU ET AL: 'Structure of the type 5 capsular polysaccharide of Staphylococcus aureus' CARBOHYDRATE RESEARCH vol. 201, 1990 - 1990, pages 285 - 297
- MCKENNEY ET AL: 'The ica locus of staphylococcus epidermididis encodes production of the capsular polysaccharide/adhesin' INFECTION AND IMMUNITY vol. 66, no. 10, 1998 - 1998, pages 4711 - 4720
- WICKEN; KNOX: 'Characterisation of Group N Streptococcus Lipoteichoic acid' INFECTION AND IMMUNITY vol. 11, no. 5, 1975,
- GEHORN ET AL: 'Determination of the gram positive bacterial content of soils and sedimentst by analysis of teichoic acid componients' JOURNAL OF BIOLOGICAL METHODS vol. 2, 1984, pages 165 - 176

## Description

### Field Of The Invention

The present invention relates to polysaccharide compositions useful for inducing immunity for the prevention of *staphylococcal* infections. The invention also relates to methods of making and using diagnostic kits and for inducing active and passive immunity using the polysaccharide material and antibodies thereto. In particular, the polysaccharide is a capsular polysaccharide/adhesin.

### Background Of The Invention

*Staphylococci* are gram-positive bacteria which normally inhabit and colonize the skin and mucus membranes of humans. If the skin or mucus membrane becomes damaged during surgery or other trauma, the *staphylococci* may gain access to internal tissues causing infection to develop. If the *staphylococci* proliferate locally or enter the lymphatic or blood system, serious infectious complications such as those associated with *staphylococcal bacteremia* may result. Complications associated with *staphylococcal bacteremia* include septic shock, endocarditis, arthritis, osteomyelitis, pneumonia, and abscesses in various organs.

*Staphylococci* include both coagulase positive organisms that produce a free coagulase and coagulase negative organisms that do not produce this free coagulase. *Staphylococcus aureus* is the most common coagulase-positive form of *staphylococci. S. aureus* generally causes infection at a local site, either extravascular or intravascular, which ultimately may result in *bacteremia. S. aureus* is also a leading cause of acute osteomyelitis and causes a small number of *staphylococcal* pneumonia infections. Additionally, *S. aureus* is responsible for approximately 1-9% of the cases of bacterial meningitis and 10-15% of brain abscesses. There are at least twenty-one known species of coagulase-negative *staphylococci,* including *S. epidermidis, S. saprophyticus, S. hominis, S. warneri, S. haemolyticus, S. saprophiticus, S. cohnii, S. xylosus, S. simulans,* and *S*. *capitis. S. epidermidis* is the most frequent infection-causing agent associated with intravenous access devices and the most frequent isolate in primary nosocomial *bacteremias. S. epidermidis* is also associated with prosthetic valve endocarditis.

*Staphylococcus* is also a common source of bacterial infection in animals. For instance, *staphylococcal* mastitis is a common problem in ruminants including cattle, sheep, and goats. The disease is generally treated with antibiotics to reduce the infection but the treatment is a costly procedure and still results in a loss of milk production. The most effective vaccines identified to date are live, intact *S. aureus* vaccines administered subcutaneously. The administration of live vaccines, however, is associated with the risk of infection. For that reason, many researchers have attempted to produce killed *S*. *aureus* vaccines and/or to isolate capsular polysaccharides or cell wall components which will induce immunity to *S*. *aureus.* None of these attempts, however, has been successful.

*S. aureus* includes a cell wall component composed of a peptidoglycan complex which enables the organism to survive under unfavorable osmotic conditions and also includes a unique teichoic acid linked to the peptidoglycan. External to the cell wall a thin polysaccharide capsule coats most isolates of *S*. *aureus.* This serologically distinct capsule can be used to serotype various isolates of *S. aureus.* Many of the clinically significant isolates have been shown to include two capsular types, CP5 and CP8.

Type CP5 has the following chemical structure:
→4)-β-D-Man*p*NAcA3Ac-(1→4)-α-L-Fuc*p*NAc-(1→3)-β-D-Fuc*p*NAc-(1→

Type CP8 has the following chemical structure:
→3)-β-D-Man*p*NAcA4Ac-(1→3)-α-L-Fuc*p*NAc-(1→3)-β-D-Fuc*p*NAc-(1→

Studies on over 1600 *S*. *aureus* isolates showed that 93% were of either type 5 or type 8 capsular polysaccharides. Additionally, more than 80% of the *S*. *aureus* isolated from sheep, goats, and cows with mastitis and chickens with osteomyelitis have been demonstrated to include at least one of these two capsular types. Although CP5 and CP8 are structurally similar, they have been demonstrated to be immunologically distinct

### Summary Of The Invention

The present invention is a method for preparing a pure capsular polysaccharide/ adhesin as defined in claim 1. The invention comprises a polysaccharide comprising a polysaccharide/adhesin that is greater than 92% free of contaminants preparable according to the method of the invention. The invention encompasses a composition comprising the polysaccharide of the invention conjugated to a carrier compound.

The disclosure relates to methods and products for passive and active immunization of humans and animals against infection by coagulase-negative and coagulase-positive *staphylococci.* It has been discovered, that a true "capsule" of *S*. *aureus* extends out beyond the CP5 or CP8 layer or is produced instead of the CP5 or CP8 layer. This material, which is the capsular polysaccharide/adhesin (PS/A) antigen, has been purified to near homogeneity and the structure identified. The native PS/A antigen which can be isolated from *Staphylococci* such as *S*. *aureus* and *S. epidermis* is a high molecular weight polyglucosamine which is heavily substituted with succinic acid residues, and is referred to as poly-beta-1-6-D-N-succinylglucosamine (PNSG). The isolated and purified PS/A antigen has been found to be highly immunogenic *in vivo.*

Described is a composition of a pure PS/A having the following structure: 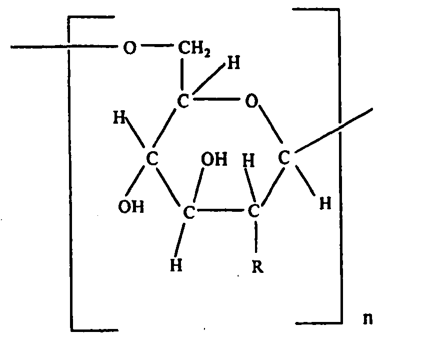 wherein n is an integer greater than or equal to 3, wherein R is selected from the group consisting of -NH-CO-(CH₂)ₘ-COOH, -NH-(CH₂)ₘ-(COOH)₂, -NH-(CH₂)ₘ-COOH, and -NH₂ wherein m is 2-5, and wherein at least 10% of the R groups are selected from the group consisting of -NH-CO-(CH₂)ₘ-COOH, -NH-(CH₂)ₘ-(COOH)₂, and -NH-(CH₂)ₘ-COOH. In other examples at least 50%, 75%, or 90% of the R groups are selected from the group consisting of -NH-CO-(CH₂)ₘ-COOH, -NH-(CH₂)ₘ-(COOH)₂, and -NH-(CH₂)ₘ-COOH. The composition is formulated as a vaccine. The PS/A is at least 95%, 97% or 99% pure. The preferred backbone linkage is a β 1-6 linkage.

The monomeric unit of PS/A can be substituted with succinate. In this example the R group is -NH-CO-CH₂-CH₂-COOH. In other examples the R group is selected from the group consisting of -NH-CO-CH₂-CH₂-CH₂-COOH, -NH-CO-CH₂-CH₂-CH₂-CH₂-COOH, -NH-CO-CH₂-CH₂-CH₂-CH₂-CH₂-COOH, -NH-CH₂-CH₂-COOH, -NH-CH₂-CH₂-CH₂-COOH, -NH-CH₂-CH₂-CH₂-CH₂-COOH, -NH-CH₂-CH₂-CH₂-CH₂-CH₂-COOH, -NH-CH₂-CH₂-(COOH)₂, -NH-CH₂-CH₂-CH₂-(COOH)₂, -NH-CH₂-CH₂-CH₂-CH₂-(COOH)₂, and -NH-CH₂-CH₂-CH₂-CH₂-CH₂-(COOH)₂. The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

The PS/A is a polymer of 3 or more repeating monomeric units and therefore may have a low molecular weight. The PS/A can have a molecular weight of greater than 25,000, or 30,000, or 100,000.

The composition may also include a carrier compound conjugated to the PS/A. When the PS/A is used as a vaccine and the PS/A has a low molecular weight it is preferred that the PS/A be conjugated to a carrier compound. In one embodiment the carrier compound is conjugated to the PS/A through a linker.

In another disclosure the PS/A is substantially free of phosphate. The PS/A is substantially free of teichoic acid.

A composition of a PS/A having the following structure is disclosed. 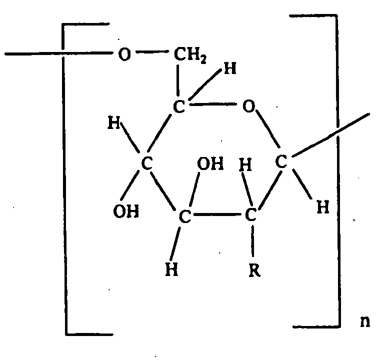 wherein n is an integer greater than or equal to 3, wherein R is selected from the group consisting of -NH-CO-(CH₂)₃₋₅-COOH, -NH-(CH₂)ₘ-(COOH)_{2'} -NH-(CH₂)ₘ-COOH, and -NH₂, wherein m is 2-5, and wherein at least 10% of the R groups are selected from the group consisting of -NH-CO-(CH₂)₃₋₅-COOH, -NH-(CH₂)ₘ-(COOH)₂, and -NH-(CH₂)ₘ-COOH. At least 50%, 75%, or 90% of the R groups can be selected from the group consisting of --NH-CO-(CH₂)₃₋₅-COOH, NH-(CH₂)ₘ-(COOH)₂, and -NH-(CH₂)ₘ-COOH. The composition can be formulated as a vaccine. The preferred backbone linkage is a β 1-6 linkage.

The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

A composition of a PS/A having the following structure is disclosed 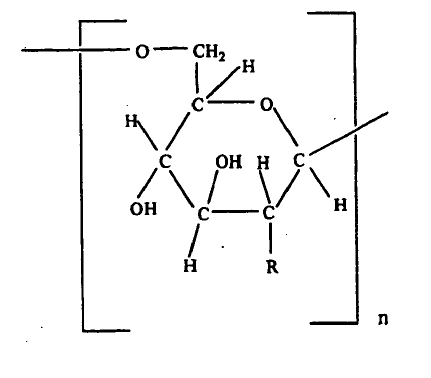 wherein n is an integer greater than or equal to 3, wherein R is selected from the group consisting of -NH-CO-CH₂-CH₂-COOH and -NH₂ and wherein between 10 and 90% of the R groups are -NH-CO-CH₂-CH₂-COOH. In other preferred embodiments between 20 and 80%, 30 and 70%, or 40 and 60% of the R groups are -NH-CO-CH₂-CH₂-COOH. The composition may be is formulated as a vaccine. The preferred backbone linkage is a β 1-6 linkage.

The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

Described is a composition of a PS/A composed of repeating monomer units wherein the monomer unit is selected from the group consisting of β-1-6 polyglucose, α-1-4 polyglucose, α-1-3 polyglucose, β-1-4 polyglucose, β-1-3 polyglucose, and a α-1-4 polyglactosamine and wherein C2 of the monomer unit is substituted with an R group selected from the group consisting of -NH-CO-(CH₂)ₘ-COOH, -NH-(CH₂)ₘ-(COOH)₂, -NH-(CH₂)ₘ-COOH, and -NH₂, wherein m is 2-5, and wherein at least 10% of the R groups are selected from the group consisting of -NH-CO-(CH₂)ₘ-COOH, -NH-(CH₂)ₘ-(COOH)₂, and -NH-(CH₂)ₘ-COOH is provided. The composition may be formulated as a vaccine.

Preferably the monomer unit is selected from the group consisting of β-1-6 polyglucose, α-1-4 polyglucose, α-1-3 polyglucose, β-1-4 polyglucose, and β-1-3 polyglucose. Preferably the polyglucose backbone is in an α-1-6 formation. In another embodiment the polyglucose backbone is in a β-1-4 formation. The polyglucose backbone may be in a β-1-3 formation.

At least 50%, 75%, or 90% of the R groups are selected from the group consisting of -NH-CO-(CH₂)ₘ-COOH, -NH-(CH₂)ₘ-(COOH)₂, and -NH-(CH₂)ₘ-COOH.

The monomeric unit of PS/A may be substituted with succinate. In this case the R group is -NH-CO-CH₂-CH₂-COOH. The R group may be selected from the group consisting of -NH-CO-CH₂-CH₂-CH₂-COOH, -NH-CO-CH₂-CH₂-CH₂-CH₂-COOH, -NH-CO-CH₂-CH₂-CH₂-CH₂-CH₂-COOH, -NH-CH₂-CH₂-COOH, -NH-CH₂-CH₂-CH₂-COOH, -NH-CH₂-CH₂-CH₂-CH₂-COOH, -NH-CH₂-CH₂-CH₂-CH₂-CH₂-COOH, -NH-CH₂-CH₂-(COOH)₂, -NH-CH₂-CH₂-CH₂-(COOH)₂, -NH-CH₂-CH₂-CH₂-CH₂-(COOH)₂, and -NH-CH₂-CH₂-CH₂CH₂-CH₂-(COOH)₂. The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

A composition of a PS/A having the following structure is disclosed 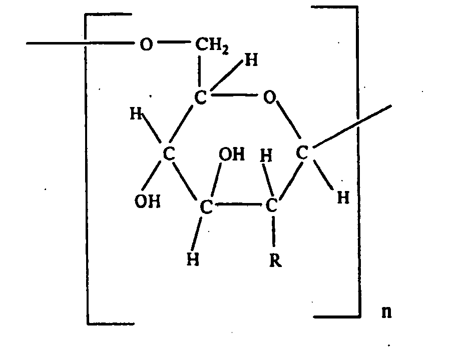 wherein n is an integer greater than or equal to 3, wherein R is selected from the group consisting of -NH-CO-(CH₂)ₘ-COOH, -NH-(CH₂)ₘ-(COOH)₂, -NH-(CH₂)ₘ-COOH, and -NH₂, wherein m is 2-5, and wherein at least 10% of the R groups are selected from the group consisting of -NH-CO-(CH₂)ₘ-COOH; -NH-(CH₂)ₘ-(COOH)₂, and -NH-(CH₂)ₘ-COOH and wherein the PS/A is substantially free of teichoic acid is provided. At least 50%, 75%, or 90% of the R groups may be selected from the group consisting of -NH-CO-(CH₂)ₘ-COOH, -NH-(CH₂)ₘ-(COOH)₂, and -NH-(CH₂)ₘ-COOH. The composition is formulated as a vaccine. The preferred backbone linkage is a β 1-6 linkage.

The monomeric unit of PS/A can be substituted with succinate. In this embodiment the R group is -NH-CO-CH₂-CH₂-COOH. In other embodiments the R group is selected from the group consisting of -NH-CO-CH₂-CH₂-CH₂-COOH, -NH-CO-CH₂-CH₂-CH₂-CH₂-COOH, NH-CO-CH₂-CH₂-CH₂-CH₂-CH₂-COOH, -NH-CH₂-CH₂-COOH, -NH-CH₂-CH₂-CH₂-COOH, -NH-CH₂-CH₂-CH₂-CH₂-COOH, -NH-CH₂-CH₂-CH₂-CH₂-CH₂-COOH, -NH-CH₂-CH₂-(COOH)₂, -NH-CH₂-CH₂-CH₂-(COOH)₂, -NH-CH₂-CH₂-CH₂-CH₂-(COOH)₂, and -NH-CH₂-CH₂-CH₂-CH₂-CH₂-(COOH)₂. The PS/A may be substituted with a single type of R group substituent or by more than one type of R group substituent. When PS/A is substituted with a single type of R group substituent the PS/A is a homo-substituted polymer. When PS/A is substituted with more than a single type of R group substituent the PS/A is a hetero-substituted polymer.

In a preferred embodiment of the method of the invention the bacterial culture is a *Staphylococcus aureus* culture. In another preferred embodiment the bacterial culture is a coagulase negative *Staphylococci* culture. Another aspect of the invention is use of the pure capsular polysaccharide/adhesin in the preparation of a medicament for producing an antibody response. In another aspect the invention is a use for inducing active immunity to infection by *staphylococci* in a subject. The use includes the step of administering to a subject an effective amount for inducing active immunity to *staphylococci* of any of the composition of the invention.

*Staphylococcus aureus,* is the most common cause of bacterial infection in hospitalized patients.

### Brief Description Of The Drawings

Figure 1 is an NMR spectra of PS/A from *S*. *aureus* strain MN8.

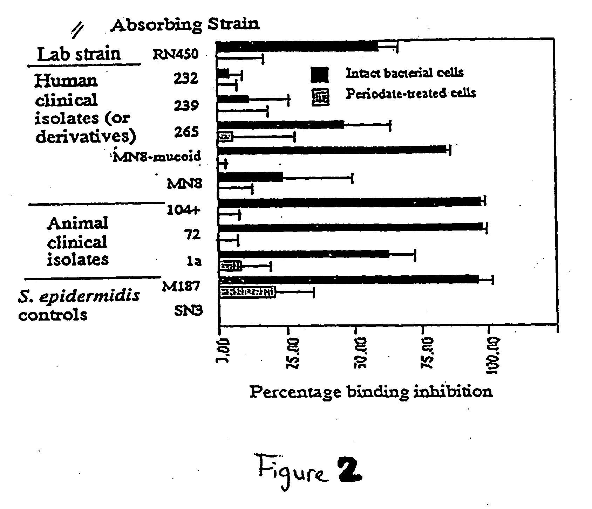

Figure 2 is a graph depicting the results of an ELISA inhibition showing the ability of various strains of *Staphylococcus aureus* grown overnight two times in brain heart infusion broth supplemented with 0.5% glucose to remove antibodies binding to PS/A antigen that has sensitized the ELISA plate (speckled bars). As a control fro specificity a separate aliquot of the same bacteria was treated with 0.4 M sodium metaperiodate to destroy the PS/A antigen on the surface of the *S*. *aureus* strains (grey bars). The percentage inhibition of binding was determined from the ratio of the optical density achieved using antibody to PS/A adsorbed with the test strain and antibody to PS/A adsorbed with a known PS/ A negative strain (*S. epidermidis* sn-3).
Figure 3 is a graph depicting the ability of a PS/A antigen to induce active immunity in mice against *S. aureus* infection.
Figure 4 is a graph depicting the ability of an anti-PS/A antibody to induce passive immunity in mice against *S*. *aureus* infection.
Figure 5 shows a photograph of a PCR fragment gel.
Figure 6 shows a photograph of a Southern blot of *ica* (intercellular adhesin locus) genes in *S*. *aureus*. PCR was performed on chromosomal DNA from controls and eight isolates of *S*. *Aureus:* (1) molecular weight markers (2) *S*. *Carnosus* TM300, negative control (3) positive control DNA from *S*. *Epidermis* RP62A *staphylococcus aureus* strains (4) Reynolds (5) MN8 (6) 5827 (7) S836 (8) Vas (9) VP (10) 265 (11) Por).

### Brief Description Of The Sequences

SEQ. ID. NO. 1 is nucleic acid sequence of the *ica* locus which has been deposited in GenBank under accession number U43366. (Prior Art)

SEQ. ID. NO. 2 is a forward primer sequence for *ica.*

SEQ. ID. NO. 3 is a backward primer sequence for *ica.*

### Detailed Description Of The Invention

The invention relates to a microcapsule from *Staphylococcal* bacteria which is useful for inducing immunity to bacterial infection and also for producing antibodies for diagnostic and therapeutic purposes. The microcapsule is a PS/A preparation which can be isolated from *Staphylococcus aureus* or coagulase-negative *staphylococci,* or can be synthesized *de novo.* PS/A has not previously been identified as being a component of the *Staphylococcus aureus* extracellular layer. Prior to the instant invention, it was demonstrated that two chemically related serologic types of capsule, termed capsular polysaccharide CP5 and CP8 were produced by 90% of *Staphylococcus aureus* strains. It was believed that these capsular polysaccharides were responsible for inducing immune recognition of *S*. *aureus.* It has been discovered that *S*. *aureus* includes a true capsule which either extends out beyond or replaces the CP5 or CP8 layer and is responsible for provoking an immune response. As demonstrated in the examples presented herein, the PS/A antigen can produce opsonic protective antibodies against *S*. *aureus* and coagulase-negative *staphylococci* when administered *in vivo.*

Prior to the invention, the inventor of the instant application identified and characterized a portion of the capsule of coagulase-negative *staphylococci,* which was PS/A (U.S. Patent No. 5,055,455, issued to Gerald B. Pier). It was found that the PS/A of coagulase negative *staphylococcus* is a component of the cell surface and biofilm layer and is involved in protecting the bacterial cell from host defenses, such as opsonophagocytosis (Kojima, Y., M. Tojo, D. A. Goldmann, T. D. Tosteson and G. B. Pier. (1990) J Infect Dis.162:435*.* Tojo, M., N. Yamashita, D. A. Goldmann and G. B. Pier. (1988) J Infect Dis. 157:713*.* Goldmann, D. A. and G. B. Pier. (1993) Clin Microbiol Rev. 6:176*.)* The chemical structure of the PS/A, however, was not identified because of the difficulty associated with purifying the isolated PS/A. It was only possible to achieve a preparation of approximately 90% purity. According to the methods of the instant invention, the PS/A antigen has been isolated and purified to achieve a greater than 92% pure PS/A preparation.

As used herein, a "pure capsular polysaccharide/adhesin" is a PS/A preparation which has been isolated or synthesized and which is greater than 92% free of contaminants. Preferably, the material is greater than 95% or even greater than 97% or 99% free of contaminants. The degree of purity of the PS/A antigen can be assessed by any means known in the art. For example, the purity can be assessed by chemical analysis assays as well as gas chromatography and nuclear magnetic resonance to verify structural aspects of the material.

One major contaminant of the prior art PS/A antigen was phosphate containing teichoic acid. The teichoic acid contamination of the prior art antigen interfered with both the chemical characterization and the immunogenicity of the antigen. The procedures used in the prior art to isolate PS/A were not sufficient to remove contaminating teichoic acid from the PS/A preparation, making the chemical characterization of the PS/A antigen impossible. The methods of the invention described herein are capable of producing an isolated PS/A antigen preparation which is substantially free of teichoic acid. A PS/A preparation that is substantially free of teichoic acid is one which has less than 1.0% phosphate. In some embodiments the PS/A preparation that is substantially free of teichoic acid is one which has less than 0.1 % phosphate. The amount of phosphate present in the sample can be assessed by any means known in the art. As described in the examples below the amount of phosphate contamination can be assessed using the methods described in Keleti, G. and W.H. Lederer, (1974) Handbook of Micromethods for the Biological Sciences Van Nostrand Reinhold Co., New York, which is hereby incorporated by reference. Briefly, the assay is performed as follows: to 100 µg of sample 100 µl of a solution made by adding together 43.5 ml of water, 6.5 ml of 70% perchloric acid (HCLO₄) and 50 ml of 20 N sulfuric acid (H₂SO₄) is added. This is heated at 95 ° C for 2 hours in a tube with a marble on top of it. The mixture is next placed in an oven at 165 ° C and heated for an additional 2 hours then cooled to room temperature. Next, one ml of reagent 5, made by the following method, is added to the sample:
Reagent 1: 1.36 grams of sodium acetate .3H₂0 dissolved in 10 ml water.
Reagent 2: 500 mg ammonium molybdate dissolved in 20 ml water.
Reagent 3: 2 ml of reagent 1, 2 ml of reagent 2 and 16 ml of water.
Reagent 4: 2 gm ascorbic acid dissolved in 20 ml water, prepared immediately prior to use.
Reagent 5: Add in an ice bath 9 ml of reagent 3 and 1 ml of reagent 4.

After adding reagent 5 the tubes are mixed throughly and the optical density read at 820 nanometers in a spectrophotometer. A standard curve consisting of sodium phosphate monobasic (range of 0.1-5 µg per tube) is used to calculate the amount of phosphate present in the test samples. (Lowry, O.H., N.R. Roberts, K.Y. Leiner, M.L. Wu and A. L. Farr., (1954), Biol. Chem. 207,1.)

The compositions of the invention are useful in a variety of different applications including *in vitro, in situ* and *in vivo* diagnosis of pathological status, such as infection. Additionally, these compositions may be used to immunize subjects *in vivo* to prevent infection. The compositions may also be used to develop antibodies and other binding peptides which are useful for the same purposes as the PS/A compositions of the invention. Thus, the invention includes methods for generating antibodies which are specific for PS/A and which can be used in the diagnosis and treatment of infectious disorders.

The antibodies may be either monoclonal antibodies or polyclonal antibodies. Polyclonal antibodies generally are raised in animals by multiple subcutaneous or intraperitoneal injections of an antigen and an adjuvant. Polyclonal antibodies to PS/A antigen can be generated by injecting the PS/A antigen alone or in combination with an adjuvant, or by injecting PS/A conjugated to a carrier compound. For instance, it may be useful to conjugate the PS/A antigen to a protein, e.g., keyhole limpet hemocyanin, serum albumin, bovine thyroglobulin, or soy bean trypsin inhibitor, that is immunogenic in the species of animal to be immunized.

Many methods are known in the art for conjugating a polysaccharide to a protein. In general, the polysaccharide should be activated or otherwise rendered amenable to conjugation, i.e., at least one moiety must be rendered capable of covalently bonding to a protein or other molecule. Many such methods are known in the art. For instance, U.S. Patent No. 4,356,170, issued to Jennings, describes the use of periodic acid to generate aldehyde groups on the polysaccharide and then performs reductive amination using cyanoborohydride. U.S. Patent No. 4,663,160, issued to Tsay et al., also used periodic acid to generate aldehyde groups but then linked the polysaccharide to a protein derivatized with a 4-12 carbon moiety (prepared in the presence of a condensing agent) with a Schiff's base reaction in the presence of a reducing agent such as cyanoborohydride. U.S. Patent No. 4,619,828, issued to Gordon, used cyanogen bromide to active the polysaccharide and then conjugated it through a spacer bridge of 4-8 carbon atoms to the protein. In U.S. Patent No. 4,808,700, issued to Anderson and Clements, a polysaccharide was modified to produce at least one reducing end using limited oxidative cleavage by periodate, hydrolysis by glycosidases, or acid hydrolysis and was conjugated to a protein through reductive amination in the presence of cyanoborohydride. U.S. Patent No. 4,711,779, issued to Porro and Costantino, described the activation of polysaccharides by introducing primary amino groups into the terminal reducing group using sodium cyanoborohydride, followed by conversion to esters in the presence of adipic acid derivatives and conjugation to a toxoid in the presence of an organic solvent, such as dimethylsulfoxide. Many other methods of conjugation are known in the art.

The carrier compound may be directly linked to the PS/A antigen or may be connected to the PS/A antigen through a linker or spacer. A polysaccharide may be coupled to a linker or a spacer by any means known in the art, for example, using a free reducing end of the polysaccharide to produce a covalent bond with a spacer or linker. A covalent bond may be produced by converting a free reducing end of a PS/A antigen into a free 1-aminoglycocide, which can subsequently be covalently linked to a spacer by acylation. (Lundquist et al., J. Carbohydrate Chem., 10:377 (1991)). Alternatively, the PS/A antigen may be covalently linked to the spacer using an N-hydroxysuccinimide active ester as activated group on the spacer. (Kochetkow, Carbohydrate Research, 146:C1 (1986)). The free reducing end of the PS/A antigen may also be converted to a lactone using iodine and potassium hydroxide. (Isebell et al., Methods of Carbohydrate Chemistry, Academic Press, New York (1962)). The lactone can be covalently linked to the spacer by means of a primary amino group on the spacer or linker. The free reducing end of the PS/A antigen may also be covalently linked to the linker or spacer using reductive amination.

The carrier compound linked to the PS/A antigen may be an immunologically active or inert protein. Proteins include, for example, plasma proteins such as serum albumin, immunoglobulins, apolipoproteins and transferrin, bacterial polypeptides such as TRPLE, β-galactosidase, polypeptides such as herpes gD protein, allergins, diphtheria and tetanus toxoids, salmonella flagellin, hemophilus pilin, hemophilus 15kDa, 28-30kDa, and 40kDa membrane proteins, *escherichia coli,* heat label enterotoxin ltb, cholera toxin, and viral proteins including rotavirus VP and respiratory syncytial virus f and g proteins. The proteins which are useful according to the invention include any protein which is safe for administration to mammals and which serves as an immunologically effective carrier protein.

To prepare the polyclonal antibodies the PS/A antigen or antigen conjugate generally is combined with Freund's complete adjuvant or other adjuvant (1 mg or µg of conjugate for rabbits or mice, respectively, in 3 volumes of Freund's) and injected intradermally at multiple sites. Approximately one month later, the animals are boosted with 1/5 - 1/10 of the original amount of antigen or antigen conjugate in Freund's complete adjuvant by subcutaneous injection at multiple sites. One to two weeks later the animals are bled, and the serum is assayed for the presence of antibody. The animals may be repeatedly boosted until the antibody titer plateaus. The animal may be boosted with the PS/A antigen alone, the PS/A antigen conjugate, or PS/A conjugated to a different carrier compound. An aggregating agent, such as alum, can also be used to enhance the immune response.

In addition to supplying a source of polyclonal antibodies, the immunized animals can be used to generate PS/A antigen specific monoclonal antibodies. As used herein the term "monoclonal antibody" refers to a homogenous population of immunoglobulins which specifically bind to an epitope (i.e. antigenic determinant) of PS/A. Monoclonal antibodies can be prepared by any method known in the art such as by immortalizing spleen cells isolated from the immunized animal by e.g., fusion with myeloma cells or by Epstein-bar-virus transformation and screening for clones expressing the desired antibody. Methods for preparing and using monoclonal antibodies are well known in the art.

Murine anti-PS/A monoclonal antibodies may be made by any of these methods utilizing PS/A as an immunogen. The following description of a method for developing an anti-PS/A monoclonal antibody is exemplary and is provided for illustrative purposes only. Balb/c mice are immunized intraperitoneally with approximately 75-100 µg of purified PS/A in an complete Freund's adjuvant. Booster injections of approximately 25-50 µg PS/A in incomplete Freund's are administered on approximately days 15 and 35 after the initial injection. On day 60-65, the mice receive booster injections of approximately 25 µg PS/A in the absence of adjuvant. Three days later, the mice are killed and the isolated spleen cells fused to murine myeloma NS-1 cells using polyethylene glycol by a procedure such as that described by Oi (Oi VT: Immunoglobulin-producing hybrid cell lines in Hezenberg LA (ed): selected methods in cellular biology, San Francisco, CA, Freeman, (1980)). Hybridoma cells are selected using hypoxanthine, aminopterin, and thymidine and grown in culture. Fourteen to fifteen days after fusion, hybridoma cells producing anti-PS/A monoclonal antibodies are identified using a solid-phase radioimmunoassay by capturing anti-PS/A antibodies from conditioned media with immobilized goat anti-mouse IgG followed by quantitation of specifically bound ¹²⁵I-labeled PS/A. Hybridomas testing positive for antibodies against PS/A are subcloned by limiting dilution and retested. Ascites for the hybridomas is then prepared in pristane-primed BALB/c mice by injecting approximately 1 x 10⁶ cells/mouse. Concentrates enriched in the selected monoclonal antibodies are produced from ascites fluid by gel filtration on S-200 and concentrated with (NH₄)SO₄. The pellets are dissolved in an appropriate storage solution such as 50% glycerol/H₂O and are stored at 4°C.

An "anti-PS/A antibody" as used herein includes humanized antibodies and antibody fragments as well as intact monoclonal and polyclonal antibodies.

The anti-PS/A antibody may be an intact humanized anti-PS/A monoclonal antibody in an isolated form or in a pharmaceutical preparation. A "humanized monoclonal antibody" as used herein is a human monoclonal antibody or functionally active fragment thereof having human constant regions and a PS/A binding region from a mammal of a species other than a human.

Human monoclonal antibodies may be made by any of the methods known in the art, such as those disclosed in US Patent No. 5,567,610, issued to Borrebaeck et al., US Patent No. 565,354, issued to Ostberg, US Patent No. 5,571,893, issued to Baker et al, Kozber, J. Immunol. 133: 3001 (1984), Brodeur, et al., Monoclonal Antibody Production Techniques and Applications, p. 51-63 (Marcel Dekker, Inc, new York, 1987), and Boerner el al., J. Immunol., 147: 86-95 (1991). In addition to the conventional methods for preparing human monoclonal antibodies, such antibodies may also be prepared by immunizing transgenic animals that are capable of producing human antibodies (e.g., Jakobovits et al., PNAS USA, 90: 2551 (1993), Jakobovits et al., Nature, 362: 255-258 (1993), Bruggermann et al., Year in Immuno., 7:33 (1993) and US Patent No. 5,569,825 issued to Lonberg).

The following examples of methods for preparing humanized monoclonal antibodies that interact with PS/A are exemplary and are provided for illustrative purposes only. Humanized monoclonal antibodies, for example, may be constructed by replacing the non-CDR regions of a non-human mammalian antibody with similar regions of human antibodies while retaining the epitopic specificity of the original antibody. For example, non-human CDRs and optionally some of the framework regions may be covalently joined to human FR and/or Fc/pFc' regions to produce a functional antibody. There are entities in the United States which will synthesize humanized antibodies from specific murine antibody regions commercially, such as Protein Design Labs (Mountain View California).

European Patent Application 0239400, provides an exemplary teaching of the production and use of humanized monoclonal antibodies in which at least the CDR portion of a murine (or other non-human mammal) antibody is included in the humanized antibody. Briefly, the following methods are useful for constructing a humanized CDR monoclonal antibody including at least a portion of a mouse CDR. A first replicable expression vector including a suitable promoter operably linked to a DNA sequence encoding at least a variable domain of an Ig heavy or light chain and the variable domain comprising framework regions from a human antibody and a CDR region of a murine antibody is prepared. Optionally a second replicable expression vector is prepared which includes a suitable promoter operably linked to a DNA sequence encoding at least the variable domain of a complementary human Ig light or heavy chain respectively. A cell line is then transformed with the vectors. Preferably the cell line is an immortalized mammalian cell line of lymphoid origin, such as a myeloma, hybridoma, trioma, or quadroma cell line, or is a normal lymphoid cell which has been immortalized by transformation with a virus. The transformed cell line is then cultured under conditions known to those of skill in the art to produce the humanized antibody.

As set forth in European Patent Application 0239400 several techniques are well known in the art for creating the particular antibody domains to be inserted into the replicable vector. (Preferred vectors and recombinant techniques are discussed in greater detail below.) For example, the DNA sequence encoding the domain may be prepared by oligonucleotide synthesis. Alternatively a synthetic gene lacking the CDR regions in which four framework regions are fused together with suitable restriction sites at the junctions, such that double stranded synthetic or restricted subcloned CDR cassettes with sticky ends could be ligated at the junctions of the framework regions. Another method involves the preparation of the DNA sequence encoding the variable CDR containing domain by oligonucleotide site-directed mutagenesis. Each of these methods is well known in the art. Therefore, those skilled in the art may construct humanized antibodies containing a murine CDR region without destroying the specificity of the antibody for its epitope.

Humanized antibodies have particular clinical utility in that they specifically recognize PS/A but will not evoke an immune response in humans against the antibody itself. In a most preferred embodiment, a murine CDR is grafted into the framework region of a human antibody to prepare the " humanized antibody." See, e.g., L. Riechmann et al., Nature 332, 323 (1988); M. S. Neuberger et al., Nature 314, 268 (1985) and EPA 0 239 400 (published Sep. 30, 1987).

PS/A binding antibody fragments are also disclosed. As is well-known in the art, only a small portion of an antibody molecule, the paratope, is involved in the binding of the antibody to its epitope (see, in general, Clark, W.R. (1986) The Experimental Foundations of Modern Immunology Wiley & Sons, Inc., New York; Roitt, I. (1991) Essential Immunology, 7th Ed., Blackwell Scientific Publications, Oxford). The pFc' and Fc regions of the antibody, for example, are effectors of the complement cascade but are not involved in antigen binding. An antibody from which the pFc' region has been enzymatically cleaved, or which has been produced without the pFc' region, designated an F(ab')₂ fragment, retains both of the antigen binding sites of an intact antibody. An isolated F(ab')₂ fragment is referred to as a bivalent monoclonal fragment because of its two antigen binding sites. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd (heavy chain variable region). The Fd fragments are the major determinant of antibody specificity (a single Fd fragment may be associated with up to ten different light chains without altering antibody specificity) and Fd fragments retain epitope-binding ability in isolation.

The terms Fab, Fc, pFc', F(ab')₂ and Fv are employed with either standard immunological meanings [Klein, Immunology (John Wiley, New York, NY, 1982); Clark, W.R. (1986) The Experimental Foundations of Modern Immunology (Wiley & Sons, Inc., New York); Roitt, I. (1991) Essential Immunology, 7th Ed., (Blackwell Scientific Publications, Oxford)]. Well-known functionally active antibody fragments include but are not limited to F(ab')₂, Fab, Fv and Fd fragments of antibodies. These fragments which lack the Fc fragment of intact antibody, clear more rapidly from the circulation, and may have less non-specific tissue binding than an intact antibody (Wahl et al., J. Nucl. Med. 24:316-325 (1983)). For example, single-chain antibodies can be constructed in accordance with the methods described in U.S. Patent No. 4,946,778 to Ladner et al. Such single-chain antibodies include the variable regions of the light and heavy chains joined by a flexible linker moiety. Methods for obtaining a single domain antibody ("Fd") which comprises an isolated variable heavy chain single domain, also have been reported (see, for example, Ward et al., Nature 341:644-646 (1989), disclosing a method of screening to identify an antibody heavy chain variable region (V_{H} single domain antibody) with sufficient affinity for its target epitope to bind thereto in isolated form). Methods for making recombinant Fv fragments based on known antibody heavy chain and light chain variable region sequences are known in the art and have been described, e.g., Moore et al., US Patent No. 4,462,334. Other references describing the use and generation of antibody fragments include e.g., Fab fragments (Tijssen, Practice and Theory of Enzyme Immunoassays (Elsevieer, Amsterdam, 1985)), Fv fragments (Hochman et al., Biochemistry 12: 1130 (1973); Sharon et al., Biochemistry 15: 1591 (1976); Ehrilch et al., U.S. Patent No. 4,355,023) and portions of antibody molecules (Audilore-Hargreaves, U.S. patent No. 4,470,925). Thus, those skilled in the art may construct antibody fragments from various portions of intact antibodies without destroying the specificity of the antibodies for the PS/A epitope.

The antibody fragments may also encompass "humanized antibody fragments." As one skilled in the art will recognize, such fragments could be prepared by traditional enzymatic cleavage of intact humanized antibodies. If, however, intact antibodies are not susceptible to such cleavage, because of the nature of the construction involved, the noted constructions can be prepared with immunoglobulin fragments used as the starting materials; or, if recombinant techniques are used, the DNA sequences, themselves, can be tailored to encode the desired "fragment" which, when expressed, can be combined *in vivo* or *in vitro,* by chemical or biological means, to prepare the final desired intact immunoglobulin fragment.

Antibody fragments and other PS/A binding polypeptides having binding specificity for PS/A for the diagnostic methods of the invention set forth below also are embraced by the invention. Several routine assays may be used to easily identify such peptides. Screening assays for identifying peptides of the invention are performed for example, using phage display procedures such as those described in Hart, et al., J. Biol. Chem. 269:12468 (1994). Hart et al. report a filamentous phage display library for identifying novel peptide ligands for mammalian cell receptors. In general, phage display libraries using, e.g., M13 or fd phage, are prepared using conventional procedures such as those described in the foregoing reference. The libraries display inserts containing from 4 to 80 amino acid residues. The inserts optionally represent a completely degenerate or a biased array of peptides. Ligands that bind selectively to PS/A are obtained by selecting those phages which express on their surface a ligand that binds to PS/A. These phages then are subjected to several cycles of reselection to identify the peptide ligand-expressing phages that have the most useful binding characteristics. Typically, phages that exhibit the best binding characteristics (e.g., highest affinity) are further characterized by nucleic acid analysis to identify the particular amino acid sequences of the peptides expressed on the phage surface and the optimum length of the expressed peptide to achieve optimum binding to PS/A. Alternatively, such peptide ligands can be selected from combinatorial libraries of peptides containing one or more amino acids. Such libraries can further be synthesized which contain non-peptide synthetic moieties which are less subject to enzymatic degradation compared to their naturally-occurring counterparts.

To determine whether a peptide binds to PS/A any known binding assay may be employed. For example, the peptide may be immobilized on a surface and then contacted with a labeled PS/A. The amount of PS/A which interacts with the peptide or the amount which does not bind to the peptide may then be quantitated to determine whether the peptide binds to PS/A. A surface having an anti-PS/A antibody immobilized thereto may serve as a positive control.

The compositions of the invention are useful for many *in vivo,* and *in vitro* purposes. For example, the compositions of the invention are useful for producing an antibody response, e.g., as a vaccine for active immunization of humans and animals to prevent *S*. *aureus* infection and infections caused by other species of bacteria that make the PS/A antigen; as a vaccine for immunization of humans or animals to produce anti-PS/A antibodies that can be administered to other humans or animals to prevent or treat staphylococcal infections; as an antigen to screen for important biological agents such as monoclonal antibodies capable of preventing *S*. *aureus* infection, libraries of genes involved in making antibodies, or peptide mimetics; as a diagnostic reagent for *S*. *aureus* infections and infections caused by other species of bacteria that make the PS/A antigen; and as a diagnostic reagent for determining the immunologic status of humans or animals in regard to their susceptibility to *S*. *aureus* infections and infections caused by other species of bacteria that make the PS/A antigen.

The PS/A of the invention can be used to protect a subject against infection with a bacteria which has a PS/A capsule on its surface by inducing active immunity to infection by *staphylococci* in a subject. The method is accomplished by administering to the subject an effective amount for inducing active immunity to *staphylococci* of any of the PS/A compositions of the invention.

"Active immunity" as used herein involves the introduction of an antigen into a subject such that the antigen causes differentiation of some lymphoid cells into cells that produce antibody and other lymphoid cells into memory cell. The memory cells do not secrete antibodies but rather incorporate the antibodies into their membrane in order to sense antigen if it is administered to the body again.

The method is useful for inducing immunity to infection by *staphylococci. "Staphylococci"* as used herein refers to all *staphylococcal* bacterial species expressing a PS/A containing capsule. Bacteria which are classified as *staphylococci* are well known to those of skill in the art and are described in the microbiology literature. *Staphylococci* expressing a PS/A containing capsule include but are not limited *Staphylococcus epidermidis* (including RP62A (ATCC Number: 35984), RP12 (ATCC Number: 35983), and M187), *Staphylococcus aureus* (including RN4220 (pCN27) and MN8 mucoid), and strains such as *Staphylococcus carnosus* transformed with the genes in the *ica* locus (including TM300 (pCN27)). Other bacterial strains expressing a PS/A containing capsule can easily be identified by those of skill in the art. For instance a *staphylococcal* bacteria which expresses the *ica* locus will express a PS/A containing capsule. One of ordinary skill in the art can easily screen for the expression of mRNA or protein related of the *ica* locus since the nucleic acid sequence of the *ica* locus is known (SEQ ID NO. 1 and originally described in Heilmann, C., O. Schweitzer, C. Gerke, N. Vanittanakom, D. Mack and F. Gotz (1996) Molecular basis of intercellular adhesion in the biofilm-forming Staphylococcus epidermidis. Molec. Microbiol. 20:1083*.)* Although the Heilmann publication describes the nucleic acid sequence of the *ica* locus, the publication erroneously states that this locus encodes production of the PIA antigen. It was discovered according to the instant invention that *ica* actually encodes for proteins involved in the production of PS/A. Bacterial strains expressing a PS/A containing capsule also can be identified by immunoelectron microscopy (or other immunoassay) using anti-PS/A antibodies to detect the presence of the capsule on the surface of the bacteria as described in Example 6 below. Additionally the capsule of bacterial strains can be isolated as described in Example 1 and analyzed using liquid chromatography and mass spectroscopy as described in Example 4 below.

A "subject" as used herein is a warm-blooded mammal and includes, for instance, humans, primates, horses, cows, swine, goats, sheep, chicken, dogs, and cats.

The PS/A of the invention may be used for producing a medicament for of inducing an immune response to an antigen but are especially adapted to induce active immunization against systemic infection caused by *staphylococci* in a subject capable of inducing an immune response and at risk of developing a *staphylococcal* infection. A subject capable of inducing an immune response and at risk of developing a *staphylococcal* infection is a mammal possessing a normal healthy immune system that is at risk of being exposed to environmental *staphylococci.* For instance, a hospitalized patient which is not otherwise immunocompromised is at risk of developing staphylococcal infection as a result of exposure to the bacteria in the hospital environment. In particular high risk populations for developing infection by *S. aureus* include, for example, renal disease patients on dialysis, and individuals undergoing high risk surgery. High risk populations for developing infection by *S*. *epidermidis* include, for example, patients with indwelling medical devices because clinical isolates are often highly adherent to plastic surfaces as a result of their extracellular material referred to as biofilm or slime.

An "effective amount for inducing an antibody response" as used herein is an amount of PS/A which is sufficient to (i) assist the subject in producing its own immune protection by e.g. inducing the production of anti-PS/A antibodies in the subject, inducing the production of memory cells, inducing a cytotoxic lymphocyte reaction etc. and/or (ii) prevent infection by *staphylococci* from occurring in a subject which is exposed to *staphylococci*.

One of ordinary skill in the art can assess whether an amount of PS/A is sufficient to induce active immunity by routine methods known in the art. For instance the ability of a specific antigen to produce antibody in a mammal can be assessed by screening the PS/A antigen in a mouse or other subject.

Prior to the instant invention it was not known that *staphylococcus aureus* expresses PS/A on its surface. It was believed that CP5 and CP8 made up the external capsule of *staphylococcus aureus* and that these capsules might be effective antigens for inducing immunity. It was discovered according to the invention, however, that PS/A is useful as an antigen in inducing immunity active immunity to infection by *staphylococcus aureus.*

"Passive immunity" as used herein involves the administration of antibodies to a subject, wherein the antibodies are produced in a different subject (including subjects of the same and different species), such that the antibodies attach to the surface of the bacteria and cause the bacteria to be phagocytized.

Anti-PS/A antibody may be administered to any subject at risk of developing a *staphylococcal* infection to induce passive immunity. The anti-PS/A antibody can even be administered to a subject that is incapable of inducing an immune response to an antigen. Although vaccination with a PS/A antigen might not be effective in high risk immunocompromised subjects, these subjects will benefit from treatment with antibody preparations raised against *S. aureus.* A subject that is incapable of inducing an immune response is an immunocompromised subject (e.g. patient undergoing chemotherapy, AIDS patient, etc.) or a subject that has not yet developed an immune system (e.g. preterm neonate). The anti-PS/A antibody may be administered to a subject at risk of developing a *staphylococcal* infection to prevent the infectious agent from multiplying in the body or to kill the infectious agent. The anti-PS/A antibody may also be administered to a subject who already has an infection caused by *staphylococci* to prevent the infectious agent from multiplying in the body or to kill the infectious agent.

The anti-PS/A antibody may be administered to the subject in an effective amount for inducing an immune response to *staphylococcus aureus.* An "effective amount for inducing an immune response to *staphylococcus aureus"* as used herein is an amount of PS/A which is sufficient to (i) prevent infection by *staphylococci* from occurring in a subject which is exposed to *staphylococci;* (ii) inhibit the development of infection, i.e., arresting or slowing its development; and/or (iii) relieve the infection, i.e. eradication of the bacteria causing the infection.

Using routine procedures known to those of ordinary skill in the art, one can determine whether an amount of anti-PS/A antibody is an "effective amount for inducing an immune response to *staphylococcus aureus"* in an *in vitro* opsonization assay which is predictive of the degree of opsonization of an antibody. An antibody which opsonizes a *staphylococcal* bacteria is one which when added to a sample of *staphylococcal* bacteria causes phagocytosis of the bacteria. An opsonization assay may be a colorimetric assay, a chemiluminescent assay, a fluorescent or radiolabel uptake assay" a cell mediated bactericidal assay or other assay which measures the opsonic potential of a material. For instance, the following opsonization assay may be used to determine an effective amount of anti-PS/A antibody. The anti-PS/A antibody is incubated with an *staphylococcal* bacteria and a eukaryotic phagocytic cell and optionally complement proteins. The opsonic ability of the anti-PS/A antibody is determined based on the amount of *staphylococci* that remain after incubation. This can be accomplished by comparing the number of surviving *staphylococci* between two similar assays, only one of which includes opsonizing immunoglobulin or by measuring the number of viable *staphylococci* before and after the assay. A reduction in the number of *staphylococci* indicates opsonization.

Described are methods for inducing passive immunization to coagulase negative *staphylococci* in a subject by administering to a subject an effective amount for inducing opsonization of coagulase negative *staphylococci* of an anti-PS/Aₚᵤᵣₑ antibody. Although antibodies directed to PS/A isolated from coagulase negative *staphylococci* have been developed in the prior art and used to induce passive immunity to coagulase negative *staphylococci,* anti-PS/Aₚᵤᵣₑ antibodies have not previously been used for this purpose.

Subjects having a high risk of developing infection by *S*. *epidermidis* include, for example, preterm neonates, patients undergoing chemotherapy, and other patients with indwelling medical devices. Clinical isolates are often highly adherent to plastic surfaces because they elaborate an extracellular material referred to as biofilm or slime.

When PS/A antigen is used to prevent bacterial infection, it is formulated as a vaccine. A suitable carrier media for formulating a vaccine includes sodium phosphate-buffered saline (pH 7.4) or 0.125 M aluminum phosphate gel suspended in sodium phosphate-buffered saline at pH 6 and other conventional media. Generally, vaccines contain from about 5 to about 100 µg, preferably about 10-50 µg of antigen are suitable to elicit effective levels of antibody against the PS/A antigen in warm-blooded mammals. When administered as a vaccine the PS/A can optionally include an adjuvant.

The term "adjuvant" is intended to include any substance which is incorporated into or administered simultaneously with the PS/A of the invention which potentiates the immune response in the subject. Adjuvants include aluminum compounds, e.g., gels, aluminum hydroxide and aluminum phosphate, and Freund's complete or incomplete adjuvant (in which the PS/A antigen is incorporated in the aqueous phase of a stabilized water in paraffin oil emulsion). The paraffin oil may be replaced with different types of oils, e.g., squalene or peanut oil. Other materials with adjuvant properties include BCG (attenuated *Mycobacterium tuberculosis),* calcium phosphate, levamisole, isoprinosine, polyanions (e.g., poly A:U), lentinan, pertussis toxin, lipid A, saponins and peptides, e.g. muramyl dipeptide. Rare earth salts, e.g., lanthanum and cerium, may also be used as adjuvants. The amount of adjuvants depends on the subject and the particular PS/A antigen used and can be readily determined by one skilled in the art without undue experimentation.

In general, when administered for therapeutic purposes, the formulations of the invention are applied in pharmaceutically acceptable solutions. Such preparations may routinely contain pharmaceutically acceptable concentrations of salt, buffering agents, preservatives, compatible carriers, adjuvants, and optionally other therapeutic ingredients.

The compositions of the invention may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof and are not excluded from the scope of the invention. Such pharmacologically and pharmaceutically acceptable salts include, but are not limited to, those prepared from the following acids: hydrochloric, hydrobromic, sulphuric, nitric, phosphoric, maleic, acetic, salicyclic, p-toluene sulphonic, tartaric, citric, methane sulphonic, formic, malonic, succinic, naphthalene-2-sulphonic, and benzene sulphonic. Also, pharmaceutically acceptable salts can be prepared as alkaline metal or alkaline earth salts, such as sodium, potassium or calcium salts of the carboxylic acid group.

Suitable buffering agents include: acetic acid and a salt (1-2% WN); citric acid and a salt (1-3% WN); boric acid and a salt (0.5-2.5% WN); and phosphoric acid and a salt (0.8-2% W/V). Suitable preservatives include benzalkonium chloride (0.003-0.03% WN); chlorobutanol (0.3-0.9% WN); parabens (0.01-0.25% WN) and thimerosal (0.004-0.02% WN).

The present invention provides pharmaceutical compositions, for medical use, which comprise PS/A of the invention together with one or more pharmaceutically acceptable carriers and optionally other therapeutic ingredients. The term "pharmaceutically-acceptable carrier" as used herein, and described more fully below, means one or more compatible solid or liquid filler, dilutants or encapsulating substances which are suitable for administration to a human or other animal. In the present invention, the term "carrier" denotes an organic or inorganic ingredient, natural or synthetic, with which the active ingredient is combined to facilitate the application. The components of the pharmaceutical compositions also are capable of being commingled with the PS/A antigens of the present invention, and with each other, in a manner such that there is no interaction which would substantially impair the desired pharmaceutical efficiency.

Compositions suitable for parenteral administration conveniently comprise a sterile aqueous preparation of the polysaccharide, which can be isotonic with the blood of the recipient. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono or di-glycerides. In addition, fatty acids such as oleic acid find use in the preparation of injectables. Carrier formulations suitable for subcutaneous, intramuscular, intraperitoneal, intravenous, etc. administrations may be found in Remington's Pharmaceutical Sciences, Mack Publishing Company, Easton, PA.

The preparations of the invention are administered in effective amounts. An effective amount, as discussed above, is that amount of a PS/A antigen that will alone, or together with further doses, induce active immunity or opsonization of the infectious bacteria, respectively. It is believed that doses ranging from 1 nanogram/kilogram to 100 milligrams/kilogram, depending upon the mode of administration, will be effective. The preferred range is believed to be between 500 nanograms and 500 micrograms/kilogram, and most preferably between 1 microgram and 100 micrograms/kilograms. The absolute amount will depend upon a variety of factors including whether the administration is performed on a high risk subject not yet infected with the bacteria or on a subject already having an infection, the concurrent treatment, the number of doses and the individual patient parameters including age, physical condition, size and weight. These are factors well known to those of ordinary skill in the art and can be addressed with no more than routine experimentation. It is preferred generally that a maximum dose be used, that is, the highest safe dose according to sound medical judgment.

Multiple doses of the pharmaceutical compositions of the invention are contemplated. Generally immunization schemes involve the administration of a high dose of an antigen followed by subsequent lower doses of antigen after a waiting period of several weeks. Further doses may be administered as well. The dosage schedule for passive immunization would be quite different with more frequent administration if necessary. Any regimen that results in an enhanced immune response to bacterial infection and/or subsequent protection from infection may be used. Desired time intervals for delivery of multiple doses of a particular PS/A can be determined by one of ordinary skill in the art employing no more than routine experimentation.

A variety of administration routes are available. The particular mode selected will depend, of course, upon the particular PS/A selected, the particular condition being treated and the dosage required for therapeutic efficacy. The methods of this invention, generally speaking, may be practiced using any mode of administration that is medically acceptable, meaning any mode that produces effective levels of an immune response without causing clinically unacceptable adverse effects. Preferred modes of administration are parenteral routes. The term "parenteral" includes subcutaneous injections, intravenous, intramuscular, intraperitoneal, intra sternal injection or infusion techniques.

The compositions may conveniently be presented in unit dosage form and may be prepared by any of the methods well known in the art of pharmacy. All methods include the step of bringing the active PS/A into association with a carrier which constitutes one or more accessory ingredients. In general, the compositions are prepared by uniformly and intimately bringing the polymer into association with a liquid carrier, a finely divided solid carrier, or both, and then, if necessary, shaping the product. The polymer may be stored lyophilized.

Other delivery systems can include time-release, delayed release or sustained release delivery systems. Such systems can avoid repeated administrations of the polysaccharides of the invention, increasing convenience to the subject and the physician. Many types of release delivery systems are available and known to those of ordinary skill in the art. They include polymer based systems such as polylactic and polyglycolic acid, polyanhydrides and polycaprolactone; nonpolymer systems that are lipids including sterols such as cholesterol, cholesterol esters and fatty acids or neutral fats such as mono-, di and triglycerides; hydrogel release systems; silastic systems; peptide based systems; wax coatings, compressed tablets using conventional binders and excipients, partially fused implants and the like. Specific examples include, but are not limited to: (a) erosional systems in which the polysaccharide is contained in a form within a matrix, found in U.S. Patent Nos. 4,452,775 (Kent); 4,667,014 (Nestor et al.); and 4,748,034 and 5,239,660 (Leonard) and (b) diffusional systems in which an active component permeates at a controlled rate through a polymer, found in U.S. Patent Nos. 3,832,253 (Higuchi et al.) and 3,854,480 (Zaffaroni). In addition, a pump-based hardware delivery system can be used, some of which are adapted for implantation.

The PS/A antigens of the invention may be delivered in conjunction with another anti-bacterial antibiotic drug or in the form of cocktails with other bacterial antigens or antibodies. An anti-bacterial antibiotic cocktail is a mixture of any of a composition useful according to this invention with an anti-bacterial antibiotic drug. The use of antibiotics in the treatment of bacterial infection is routine. The use of antigens for inducing active immunization and antibodies to induce passive immunization is also routine. In this embodiment, a common administration vehicle (e.g., tablet, implant, injectable solution, etc.) could contain both the composition useful in this invention and the anti-bacterial antibiotic drug and/or antigen/antibody. Alternatively, the anti-bacterial antibiotic drug and/or antigen/antibody can be separately dosed.

Anti-bacterial antibiotic drugs are well known and include: penicillin G, penicillin V, ampicillin, amoxicillin, bacampicillin, cyclacillin, epicillin, hetacillin, pivampicillin, methicillin, nafcillin, oxacillin, cloxacillin, dicloxacillin, flucloxacillin, carbenicillin, ticarcillin, avlocillin, mezlocillin, piperacillin, amdinocillin, cephalexin, cephradine, cefadoxil, cefaclor, cefazolin, cefuroxime axetil, cefamandole, cefonicid, cefoxitin, cefotaxime, ceftizoxime, cefinenoxine, ceftriaxone, moxalactam, cefotetan, cefoperazone, ceftazidme, imipenem, clavulanate, timentin, sulbactam, neomycin, erythromycin, metronidazole, chloramphenicol, clindamycin, lincomycin, vancomycin, trimethoprim-sulfamethoxazole, aminoglycosides, quinolones, tetracyclines and rifampin. (See Goodman and Gilman's, Pharmacological Basics of Therapeutics, 8th Ed., 1993, McGraw Hill Inc.)

Other polysaccharide antigens and antibodies are well known in the art. For instance, the following polysaccharide antigens and/or antibodies thereto can be administered in conjunction with the PS/A antigen and /or antibody: *Salmonella typhi* capsule Vi antigen (Szu, S.C., X. Li, A.L. Stone and J.B. Robbins, Relation between structure and immunologic properties of the Vi capsular polysaccharide, Infection and Immunity. 59:4555-4561 (1991)); *E. Coli* K5 capsule (Vann, W., M.A. Schmidt, B. Jann and K. Jann, The structure of the capsular polysaccharide (K5 antigen) of urinary tract infective Escherichia coli, 010:K5:H4. A polymer similar to desulfo-heparin, European Journal of Biochemistry. 116: 359-364, (1981)); *Staphylococcus aureus* type 5 capsule (Fournier, J.-M., K. Hannon, M. Moreau, W.W. Karakawa and W.F. Vann, Isolation of type 5 capsular polysaccharide from Staphylococcus aureus, Ann. Inst. Pasteur/Microbiol. (Paris). 138: 561-567, (1987)); *Rhizobium melilori* expolysaccharide II (Glazebrook, J. and G.C. Walker, a novel expolysaccharide can function in place of the calcofluor-binding exopolysaccharide in nodulation of alfalfa by Rhizobium meliloti, Cell. 65:661-672 (1989)); *Group B streptococcus* type III (Wessels, M.R., V. Pozsgay, D.L. Kasper and H. J. Jennings, Structure and immunochemistry of an oligosaccharide repeating unit of the capsular polysaccharide of type III group B Streptococcus, Journal of Biological Chemistry. 262:8262-8267 (1987)); *Pseudomonas aeruginosa* Fisher 7 O-specific side-chain (Knirel, Y.A., N.A. Paramonov, E.V. Vinogradov, A.S. Shashkow, B.A. N.K. Kochetkov, E.S. Stanislavsky and E.V. Kholodkova, Somatic antigens of Pseudomonas aeruginosa The structure of O-specific polysaccharide chains of lipopolysaccharides of P. aeruginosa O3(Lanyi), 025 (Wokatsch) and Fisher immunotypes 3 and 7, European Journal of Biochemistry. 167:549, (1987)); *Shigella sonnei* O-specific side chain (Kenne, L., B. Lindberg and K. Petersson, Structural studies of the O-specific side-chains of the Shigella sonnei phase I lipopolysaccharide, Carbohydrate Research. 78:119-126, (1980)); *S. pneumoniae* type I capsule (Lindberg, B., Lindqvist, B., Lonngren, J., Powell, D.A., Structural studies of the capsular polysaccharide from Streptococcus pneumoniae type 1, Carbohydrate Research. 78:111-117 (1980)); and *Streptococcus pneumoniae* group antigen (Jennings, H.J., C. Lugowski and N. M. Young, Structure of the complex polysaccharide C-substance from Streptococcus pneumoniae type 1, Biochemistry. 19:4712-4719 (1980)).

Other non-polypeptide antigens and antibodies thereto are well known to the those of skill in the art and can be used in conjunction with the PS/A compositions of the invention.

PS/A antigens and antibodies are also useful in diagnostic assays for determining an immunologic status of a subject or sample or can be used as reagents in immunoassays. For instance, the antibodies may be used to detect the presence in a sample of a bacteria having a PS/A antigen on the surface. If the bacteria is present in the sample, then the antibodies may be used to treat the infected subject. The antibodies may also be used to screen bacteria for the presence of PS/A antigen and to isolate PS/A antigen and bacteria containing PS/A antigen from complex mixtures.

The above-described assays and any other assay known in the art can be accomplished by labeling the PS/A or antibodies and/or immobilizing the PS/A or antibodies on an insoluble matrix. The analytical and diagnostic methods for using PS/A and/or its antibodies use at least one of the following reagents: labeled analyte analogue, immobilized analyte analogue, labeled binding partner, immobilized binding partner, and steric conjugates. The label used can be any detectable functionality that does not interfere with the binding of analyte and its binding partner. Numerous labels are known for such use in immunoassays. For example, compounds that may be detected directly, such as fluorochrome, chemiluminescent, and radioactive labels, as well as compounds that can be detected through reaction or derivitization, such as enzymes. Examples of these types of labels include ³²P, ¹⁴C, ¹²⁵I, ³H, and ¹³¹I radioisotopes, fluorophores such as rare earth chelates or fluorescein and its derivatives, rhodamine and its derivatives, dansyl, umbelliferone, luciferases, such as firefly luciferase and bacterial luciferase (U.S. Patent No. 4,737,456), luciferin, 2,3-dihydrophthalavinediones, horseradish peroxidase (HRP), alkaline phosphatase, β-galactosidase, glucoamylase, lysozyme, saccharide oxidases, such as glucose oxidase, galactose oxidase, and glucose-6-phosphate dehydrogenase. Heterocyclic oxidases such as uricase and xanthine oxidase, coupled to an enzyme that uses hydrogen peroxide to oxidize a dye precursor such as HRP, lactoperoxidase, or microperoxidase, biotin avidin, spin labels, bacteriophage labels, and stable free radicals.

The labels can be conjugated to the PS/A or antibody by methods known to those of ordinary skill in the art. For example, U.S. Patent Nos. 3,940,475 and 3,645,090 demonstrate conjugation of fluorophores and enzymes to antibodies. Other assays which reportedly are commonly used with antigen and antibody and which can be used according to the invention include competition and sandwich assays.

### Examples

### Example 1: Purification of PS/A Antigen.

It has been discovered according to the invention that PS/A can be purified from any bacterial strain expressing the *ica* locus. Specifically, these include *Staphylococcus epidermidis, Staphylococcus aureus,* and other *Staphylococcal* strains such as *Staphylococcus carnosus* transformed with the genes in the *ica* locus. The following specific strains have been used according to the invention to purify PS/A from include *S*. *epidermidis* RP62A (ATCC Number: 35984), *S. epidermidis* RP12 (ATCC Number: 35983), *Staphylococcus epidermidis* M187, *S*. *carnosus* TM300 (pCN27), *S. aureus* RN4220 (pCN27), and S. *aureus* MN8 mucoid.

### 1. Method of purification of PS/A from coagulase-negative staphylococci containing the ica locus that encodes production ofproteins needed to synthesize PS/A:

Starting material was prepared from cultures of staphylococci expressing the *ica* genes by growing the bacteria in any growth medium supporting the growth of these staphylococcal strains. A preferred medium is a chemically defined medium (CDM) (Hussain, M., J. G. M. Hastings and P. J. White. 1991. A chemically defined medium for slime production by coagulase-negative Staphylococci. J. Med. Microbiol. 34:143*)* composed of RPMI-1640 AUTO-MOD, an RPMI-1640 preparation modified to allow sterilization by autoclaving; (Sigma Chemical Co., St. Louis, MO) as a starting base. Phenol red was omitted because it readily binds to purified PS/A. The CDM is supplemented with additional amino acids, vitamins, and nucleotides to give it a final composition similar to that described elsewhere (Hussain, M., J. G. M. Hastings and P. J. White. 1991. A chemically defined medium for slime production by coagulase-negative Staphylococci. J. Med Microbiol. 34:143*).* The medium was further supplemented with dextrose and sucrose; each was autoclaved separately and then added to a final concentration of 1%.

Cultures were inoculated with a single colony of staphylococci (Muller, E., J. Huebner, N. Gutierrez, S. Takeda, D. A. Goldmann and G. B. Pier. 1993. Isolation and characterization of transposon mutants of Staphylococcus epidermidis deficient in capsular polysaccharide/adhesin and slime. Infect Immun. 61:551) from an agar plate such as trypticase soy agar, that had been incubated overnight at 37°C. Cultures were grown with vigorous mixing at 37°C, with 2 L of O₂/min bubbled through via a sparger and the pH maintained at 7.0 by automatic addition of 5 M NaOH with a pH titrator. Cultures were grown until they ceased to need addition of NaOH to maintain the pH at 7.0 (i.e., for 48 - 72 hours).

Crude antigen was extracted from the bacterial cells directly into the culture supernatant with use of divalent cations, low pH, and heat. A preferred method is to add MgCl₂ to the culture to a final concentration of 100 mM, and to adjust the pH to 5.0. The culture was then heated and stirred. A temperature of 65°C for 90 min was satisfactory. The cell bodies were sedimented at 9000 X g for 15 min. The supernatant was concentrated to ~1000 ml via tangential-flow filtration (10,000 molecular weight (MW) cutoff filter), and the following buffer exchanges and treatments were performed while the solution was still in the filtration device: The buffer was exchanged with deionized H₂O adjusted to a pH of 5.0 (to remove excesses of Mg²⁺ ions). Next the pH was adjusted to 7.4 with 0.1 M TRIS-0.15 M NaCl, and the solution was treated for 4 hours at room temperature with 5 mg each of RNase and DNase and then for 2 hours with 5 mg of trypsin. The buffer was changed in the tangential flow device to 10 mM EDTA (pH 8.6), and sodium deoxycholate (DOC) added to a final concentration of 3%.

Another method for isolating the polysaccharide antigen of the invention involves incubating the bacteria with a strong base or acid, such as 5 Molar NaOH or HCL. The bacteria are stirred in the strong base or acid for 18-24 hours. The cell bodies are then collected by centrifugation and re-extracted two more times. The supernatant from each of the extractions is pooled and neutrilized to pH 7 using an acid or base. The resulting crude antigen suspension is dialized against deionized water for 2-24 hours. The remaining insoluble crude antigen is collected by centrifugation. The supernatant is tested for the presence of soluble antigen by known assays, such as immunological means. If the supernatant is positive for soluble antigen, the supernatant can be lyophilized and re-extracted to obtain additional crude antigen. The insoluble crude antigen is resuspended in a buffer solution of 50 mM PBS or 100 mM Tris with 150 mM NaCL.

The suspended crude antigen was treated with 5 mg each of RNase and DNase for 4 hours at 37°C and then with 5 mg of trypsin for 2 hours; the cells were sedimented, and this supernatant added to the concentrated culture supernatant as it was being treated with trypsin. The remaining cell bodies were resuspended in 10 mM EDTA/3% DOC (pH 8.6); stirred for 2 hours and sedimented a third time. The resultant supernatant was added to the concentrated culture supernatant as it was being treated with 10 mM EDTA/3% DOC (pH 8.6). Another alternative is a buffer with a pH>10. A preferred buffer is 0.4 M ammonium carbonate at a pH of 11. Finally, the pooled supernatants were concentrated via stirred-cell filtration using, for example, a 30,000 MW cutoff membrane filter). Particulate or insoluble material was sedimented by centrifugation at 2,000 x g for 30 min and the supernatant retained.

As an alternative to using the above extractions and DOC buffers to obtain dissolved, crude antigen, crude antigen was also obtained from the initial extracts of the cells by adjusting the pH of the extracts to >8.0 with base such as sodium hydroxide, adding 4 volumes of alcohol such as ethanol, recovery of the precipitated material by centrifugation, and redissolution of the precipitate in 0.1 M HCL or any buffer with a pH below 4.0.

High-molecular-weight PS/A-enriched material was next isolated from the crude slime extract by molecular sieve chromatography. A 5.0-cm X 100-cm column was packed with Sepharose CL-4B (Pharmacia, Piscataway, NJ) or a comparable molecular sieve gel and washed with 10 mM EDTA containing 1% DOC (pH 8.6). As an alternative a buffer with a pH < 4.0 was sometimes used. A preferred buffer was 0.1 m glycine-HCL at a pH of 2.0. Crude slime is applied in a volume of 10-20 ml and the PS/A containing fractions were identified by measurement of the absorption of UV light at 206 nm and immuno-dot-blot analysis using a polyclonal rabbit antiserum specific to the PS/A antigen.

Material eluting in the void-volume fractions (MW > 100,000 Daltons) of columns using DOC-based buffers was pooled and treated with proteinase K (0.1 mg/ml), which is proteolytically active in 1% DOC; the material was simultaneously concentrated to 100 ml with a stirred-cell filtration unit (30,000 MW cutoff membrane). Material eluting in the void volume of a molecular sieve column with a low pH buffer (< 4.0) was pooled, sodium phosphate dibasic added to a final concentration of at least 0.1 M and a pH of at least 8.0, and then 4 volumes of alcohol, such as 95% ethanol, was added to precipitate the PS/A antigen.

Further purification of material recovered from DOC columns used a step to destroy remnants of an alkali-labile cell wall antigen present in the preparations. The proteinase K-digested material was treated by the addition of NaOH to a final concentration of 500 mM and the mixture was stirred at 22°C for 2 hours and then neutralized with HCL. PS/A was recovered by alcohol precipitation (such as methanol added to 50% v/v) and sedimented by centrifugation at 30,000 x g for 30 min. Residual DOC and impurities were removed by numerous washes of the precipitate with pure methanol.

To remove residual teichoic acid from material recovered in the void volume of columns run using either the DOC-buffer system or low pH buffer system, the alcohol precipitates were suspended in 24% (v/v) hydrofluoric acid (HF) at 4°C for 48 hours. The HF solution was diluted in water to a concentration of 12% HF, then neutralized with NaOH and dialyzed against running water. PS/A was mostly insoluble at this point. Any remaining soluble PS/A was precipitated by the addition of methanol to 50%, the pellet was washed with pure methanol 3 to 5 times, suspended in deionized H₂O, frozen, and lyophilized.

### 2. Purification of PS/A from Staphylococcus aureus.

PS/A was also purified from *S. aureus* using either the above procedure for coagulase-negative staphylococci or a modification of the above procedure. The recovery of PS/A from *S. aureus* preferably was facilitated by using a strain that constitutively makes PS/A such as the MN8 mucoid strain. Such strains can be grown in any medium that supports their growth although a preferred medium is columbia broth. Alternately, any *S. aureus* strain with an intact *ica* locus can be grown in brain heart infusion broth supplemented with glucose at ≥ 0.25% (v/v) to produce PS/A.

Cultures were inoculated with a single colony of *Staphylococcus aureus* from any appropriate medium that had been incubated overnight at 37°C. Cultures were grown with vigorous mixing at 37°C, with 2 L of O₂/min bubbled through via a sparger and the pH maintained at 7.0 by automatic addition of 5 M NaOH with a pH titrator. Cultures were routinely grown until they cease to need addition of NaOH to maintain the pH at 7.0 (i.e., for 48 - 72 hours).

The bacterial cells were then recovered from the culture by centrifugation and resuspended in buffered saline. Then lysozyme and lysostaphin enzymes were added in concentrations of >0.1 mg/ml. This suspension was stirred for 2-24 hours at 37 °C, after which a protease was added. A preferred protease was Proteinase K at a concentration of at least 0.1 mg/ml, although other proteases such as Pronase E or trypsin can be used. The mixture was stirred at 37°C for at least 2 hours. The pH of the suspension was then lowered to below 4.0, but preferable to 2.0, using acid such as HCL. The insoluble material was removed by centrifugation and the precipitate re-extracted 3 times by resuspension in low pH (< 4.0, preferably 2.0) solutions followed by stirring for ≥10 minutes and centrifugation to remove insoluble materials. The low-pH extracts were pooled, the pH raised to above 8.0 by addition of both sodium phosphate to a concentration of ≥ 0.1 M and a base such as sodium hydroxide, and then 4 volumes of alcohol such as 95% ethanol were added. Another alternative is a buffer with a pH>10. A preferred buffer is 0.4 M ammonium carbonate at a pH of 11.

Another preferred method for isolating the polysaccharide antigen of the invention involves incubating the bacteria with a strong base or acid, such as 5 M NaOH or HCL. The bacteria are stirred in the strong base or acid for 18-24 hours. The cell bodies are then collected by centrifugation and re-extracted two more times. The supernatant from each of the extractions is pooled and neutralized to pH 7 using an acid or base. The resulting crude antigen suspension is dialyzed against deionized water for 2-24 hours. The remaining insoluble crude antigen is collected by centrifugation. The supernatant is tested for the presence of soluble antigen by known assays, such as immunological means. If the supernatant is positive for soluble antigen, the supernatant can be lyophilized and re-extracted to obtain additional crude antigen. The insoluble crude antigen is resuspended in a buffer solution of 50 mM PBS or 100 mM Tris with 150 mM NaCL. The material was then subjected to the same enzymatic treatments as described above with respect to purification method of PS/A from coagulase-negative *staphylococci.*

The insoluble material was then recovered by centrifugation, redissolved in low pH (<4.0, preferably 1.0) solutions, insoluble material was removed by centrifugation, and the high molecular weight material in the soluble fraction recovered by application to a molecular sieve column equilibrated in a buffer below pH 4.0. The preferred buffer was 0.1 M glycine-HCL at a pH of 2.0. A typical molecular sieve column has dimensions of 2.6 X 100 cm and was packed with a gel such as Sephacryl S-500 (Pharmacia). The PS/A containing fractions were identified by measurement of the absorption at 206 nm and immuno-dot-blot analysis with polyclonal rabbit antiserum to PS/A. Immunologically reactive fractions eluting in the high molecular (> 100,000) weight range were pooled, the pH raised to >8 by addition of both sodium phosphate to a concentration of ≥ 0.1 M and a base (such as sodium hydroxide) followed by addition of 4 volumes of an alcohol such as ethanol and the resultant precipitate recovered by centrifugation. The alcohol precipitates were next suspended in 24% (v/v) hydrofluoric acid (HF) at 4°C for 48 hours. The HF solution was diluted in water to a concentration of 12% HF, then neutralized with NaOH and dialyzed against running water. PS/A was mostly insoluble at this point. Any remaining soluble PS/A was precipitated by the addition of alcohol to >50% final concentration, the pellet was washed with alcohol such as 95% ethanol or pure methanol 3 to 5 times, and the pellet was suspended in deionized H₂O, frozen, and lyophilized.

### Example 2: Chemical Synthesis of PS/A antigen.

### Methods:

PS/A is chemically similar to chitin (poly-N-acetyl-glucosamine with beta-1-4 linkages) and chitosan (poly-glucosamine with beta-1-4 linkages). The N-acetyl groups of chitin were readily removed by treatment with mild base (1.0 M NaOH, 95°C, 1 hour)--to yield chitosan. The free amino group was then substituted with succinate using succinic acid added to these materials in the presence of a water soluble carbodiimide reagent that promotes the coupling of free amino groups to free carboxyl groups.

10 mg of chitosan was suspended in 1.0 of 0.1 M HCl and stirred until a gel-like suspension was obtained. One-hundred mg of succinic acid was then added followed by 75 mg of 1-ethyl-3-(3-dimethylaminopropyl) carbodiimide -HCl (EDC, Sigma) and the suspension stirred for 2 days at room temperature. The solution was then neutralized with 2 M sodium hydroxide, u bound succinic acid and free EDC removed by dialysis against water, and the succinylated chitosan lyophilized.

### Results:

The resulting material was similar to the native poly-N-succinyl glucosamine of PS/A, differing by the nature of the linkages between the individual glucosamine residues. However, we have found according to the invention that antibodies raised to PS/A will react with N-succinylated chitosan, indicating that proper chemical modification of chitin and chitosan results in a molecule with the ability to elicit antibodies to PS/A.

### Example 3: Analysis of the PS/A antigen.

Use of both base (NaOH) and hydrofluoric acid treatments to degrade contaminants significantly improve the purity of PS/A over prior art preparations previously obtained. PS/A eluted in the high molecular weight, void-volume fractions of a Sepharose 4B column (> 100,000 Da) and gave a single precipitin band in immunodiffusion, as previously shown (Tojo, M., N. Yamashita, D. A. Goldmann and G. B. Pier. 1988. Isolation and characterization of a capsular polysaccharide/adhesin from Staphylococcus epidermidis. J Infect Dis. 157:713). In addition, sensitization of ELISA wells with dilutions of PS/A down to 0.02 µg/well resulted in the binding of significantly more of a 1:500 dilution of antibody to purified PS/A than of preimmune serum. Use of 3% DOC or low pH buffers (< 4.0) to solubilize PS/A during purification was found to be important, but once the PS/A was precipitated from these solutions by methanol, it could not be resolubilized at a pH of > 4.0, even in DOC buffers. PS/A was slightly soluble (~500 µg/ml) in 50% propanol-50% butanol and in pyridine. The PS/A antigen was devoid of detectable phosphate (<.01%) (Keleti, G. and W.H. Lederer. 1974. Handbook of Micromethods for the Biological Sciences. Van Nostrand Reinhold Co., New York).

### Example 4: Anaylsis of the Chemical Properties of PS/A.

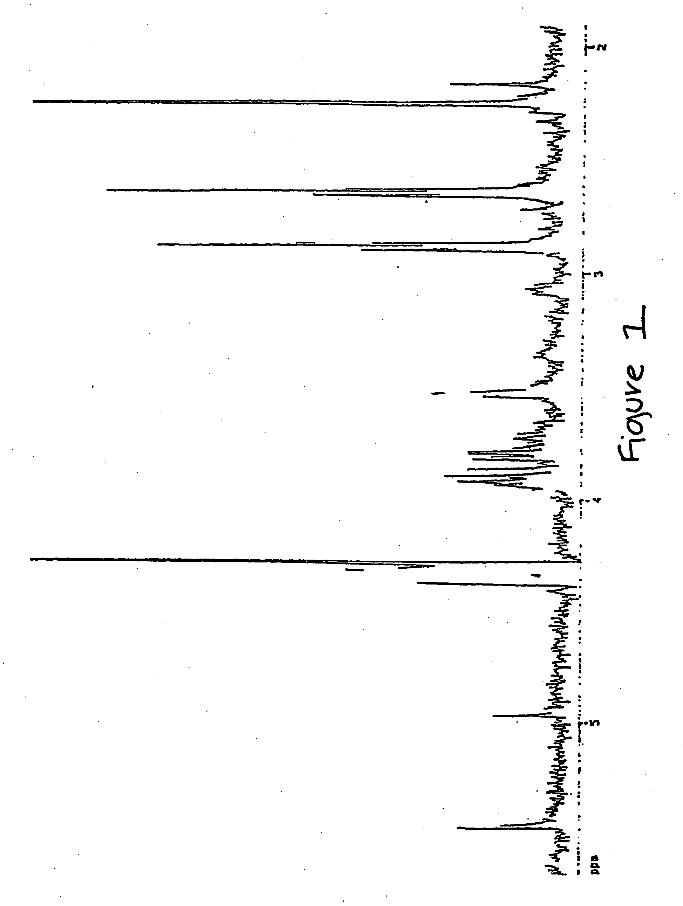

When we analyzed PS/A isolated from *S. epidermidis* M187, *S. carnosus* (pCN27) and *S. aureus* MN8 mucoid after hydrolysis in 4 M HCL acid at high temperature (95-100 °C) for 14 hours, we found that glucosamine was the single sugar component in PS/A using combinations of gas-liquid chromatography-mass spectrometry (GLC-MS) and nuclear magnetic resonance (NMR). A representative NMR spectrum is shown in Figure 1. In addition, a high quantity of succinate (about equimolar with glucosamine) was also identified in the preparations. The succinate was released from the antigens by hydrolysis in 5 M NaOH for ≥ 10 min at 95 °C, but not with 1 M NaOH under the same conditions. The lower amount of NaOH would readily release carboxyl-ester-linked succinate. Along with NMR results, this establishes the succinate as linked to the amino, and not hydroxyl, groups of glucosamine. Hydrolysis of PS/A into oligosaccharide fragments by ozone and analysis by NMR established the linkage between the sugars as a beta linkage. PS/A isolated from these strains loses serologic reactivity and chemical properties detectable by GLC-MS after treatment with sodium periodate (0.2 M for 14 h), indicating a 1-6 linkage between the glucosamine residues. Also, hydrolysis with 5 M NaOH for >5 min destroys serologic activity.

Thus the native PS/A material is a high molecular weight (>100,000 Daltons) homopolymer of N-succinyl D-glucosamine residues linked to each other in a beta 1-6 linkage. The level of substitution of the succinate groups on the amino group of the glucosamine approaches 100% in the native molecule.

### Example 5: Immunization with purified PS/A elicits antibodies reactive with the antigen.

Method: Previously described methods for eliciting antibodies to PS/A by immunization of rabbits (Kojima, Y., M. Tojo, D. A. Goldmann, T. D. Tosteson and G. B. Pier. 1990. Antibody to the capsular polysaccharide/adhesin protects rabbits against catheter related bacteremia due to coagulase-negative staphylococci. *J Infect Dis. 162:435;* Tojo, M., N. Yamashita, D. A. Goldmann and G. B. Pier. 1988. Isolation and characterization of a capsular polysaccharide/adhesin from *Staphylococcus epidermidis. J Infect Dis. 157:713;* Takeda, S., G. B. Pier, Y. Kojima, M. Tojo, E. Muller, T. Tosteson and D. A. Goldmann. 1991. Protection against endocarditis due to *Staphylococcus epidermidis* by immunization with capsular polysaccharide/adhesin. *Circulation. 84:2539*) were used to prepare antisera from the antigen isolated from *S*. *epidermidis* M187 and *S. aureus* MN8 mucoid.
**Results:** The antibodies elicited by preparations from both bacterial strains induced antibodies of comparable titers to both antigens (Table 1).

**TABLE I**

| **Binding of antibodies raised to MN8-mucoid and S. epidermidis PS/A to each others antigen in ELISA** | |
|---|---|
| **ELISA for MN8-mucoid coated PS/A plates (2µg/well)** | |
| **Anti-sera diluted 1:500** | **O.D _{405 nm}** |
| **anti-MN8 mucoid PS/A** | **0.856** |
| **anti-S. epidermidis PS/A** | **0.872** |
| **Normal rabbit sera** | **0.000** |

| **ELISA for S. epidermidis coated PS/A plates (2µg/well)** | |
|---|---|
| **Anti-sera diluted 1:500** | **O.D ₄₀₅ₙₘ** |
| **anti-MN8 mucoid PS/A** | **0.770** |
| **anti-S. epidermidis PS/A** | **0.886** |
| **Normal rabbit sera** | **0.013** |

### Example 6: Physical location of PS/A antigen on staphylococcal strains.

Methods: *S. aureus* strains MN8 mucoid and RN4220 (pCN27) were grown on trypticase soy agar, and *S. aureus* MN8 was grown in Brain Heart Infusion Broth supplemented with ≥0.25% glucose. The cells were then probed first with polyclonal rabbit antiserum to PS/A and then with gold-labeled protein A, or gold-labeled anti-rabbit IgG, and micrographs were obtained.

PS/A has been previously shown to represent the bacterial capsule for *S*. *epidermidis.* We investigated whether strains carrying the *ica* locus were encapsulated using immunoelectron microscopy. A series of immunoelectron microscopic photographs of: (a) interaction of anti-PS/A antibodies with a PS/A capsule on *S. aureus* strain MN8 mucoid; (b) negative control using normal rabbit serum (NRS) staining of *S. aureus* strain MN8 mucoid; (c) interaction of anti-PS/A antibodies with PS/A capsule on *S*. *aureus* strain RN4220(pCN27); (d) negative control using NRS staining of *S*. *aureus* strain RN4220; (e) interaction of anti-PS/A antibodies with *S*. *aureus* strain MN8 grown in brain heart infusion broth supplemented with 0.25% glucose; and (f) negative control using NRS staining of *S. aureus* strain MN8 grown in brain heart infusion broth supplemented with 0.25% glucose were taken. The micrographs demonstrate that antibodies to PS/A bind to an extracellular capsule in these strains.

### Example 7: Expression of PS/A antigen by human clinical isolates of S. aureus.

Many human clinical isolates of *S*. *epidermidis* express PS/A antigens constitutively (i.e., all of the time under a variety of growth conditions). Also, some strains of *S*. *aureus* do the same (i.e., MN8 mucoid). However most human clinical isolates do not express much PS/A when grown under normal laboratory conditions. We found that if these *S. aureus* strains were grown in brain heart infusion broth medium containing ≥0.25% glucose, then expression of PS/A could be detected by serologic means such as an ELISA inhibition assay (shown in Figure 2). In addition, we found that isolates of *S*. *aureus* that do not express detectable PS/A prior to infection of mice can be shown to produce PS/A when recovered from an infected tissue such as the kidney. Thus under specialized *in vitro* conditions or following experimental infections in animals, strains of *S*. *aureus* that otherwise do not make detectable PS/A now do so. Another medium, we have found to enhance PS/A expression *in vitro* is Staphylococcus 110 medium.

An immunoelectron microscopy photograph of a human clinical isolate (ASEAN) freshly isolated from agar to detect PS/A expression and stained with (a) anti-PS/A antibodies, or (b) NRS control was taken. We found that human clinical isolates of *S*. *aureus* express PS/A when strains were tested by transmission electron microscopy from an agar plate that was first used to detect infection in a sample of fluid or tissue. Thus if human clinical isolates were tested for PS/A expression without extensive passage of the bacteria on laboratory media then it can be shown that they make PS/A antigen.

### Example 8: Expression of PS/A antigen by animal isolates of S. aureus.

*S. aureus* is the major cause of mastitis infections in animals and causes considerable economic losses. We have also analyzed *S*. *aureus* isolates from animals (cows and sheep) for the expression of PS/A. We have found many of the isolates analyzed make the PS/A antigen, as determined by serologic testing. Out of 40 animal isolates tested, 23 had strong reactions in immuno-dot blots with antibodies to PS/A. This was detectable even though the isolates were not grown in special media to enhance production of PS/A as must be done with human isolates. Thus, unlike human clinical isolates of *S*. *aureus,* many animal isolates seem to be capable of making the PS/A constitutively.

### Example 9: Protection against S. aureus infection by antibodies to PS/A.

***Methods:*** PS/A antigen 1-100 µg (preferred 100µg/mouse) was used to immunize mice to elicit antibodies. The mice were then challenged with live *S. aureus* bacteria, either *S. aureus* strain Reynolds or strain MN8 and those with antibodies to PS/A were shown to be protected against infection.

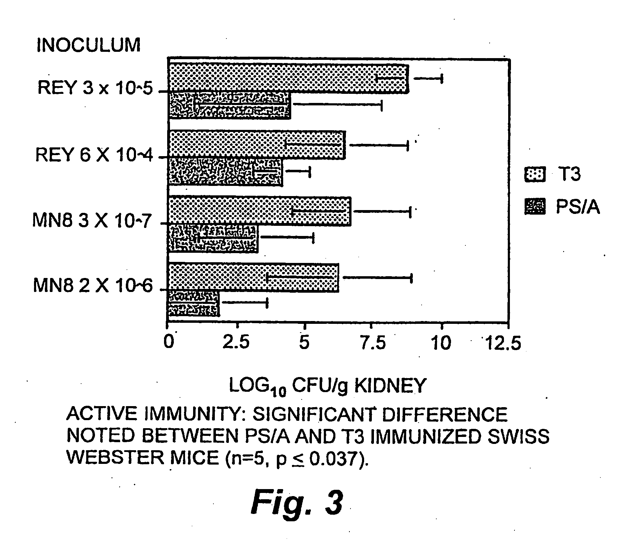

***Results:*** Actively immunizing mice with PS/A followed by intravenous challenge with *S*. *aureus* reduced the number of bacteria in the kidney's five days later when the animals were killed and tested (Figure 3).

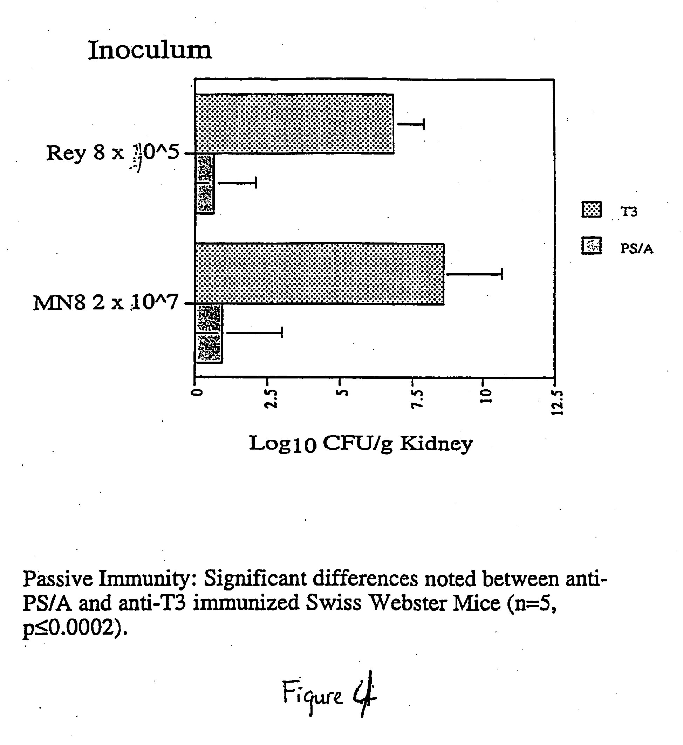

Similarly, giving mice antibodies raised in rabbits to the PS/A antigen followed by challenge of the mice with *S. aureus* strains protects the mice against *S. aureus* infection (Figure 4). Thus antibodies to PS/A protect mice against *S*. *aureus* infection. As noted above, analysis of the isolates recovered from the infected kidneys of nonimmune control mice showed that infection had induced expression of PS/A during the time of infection in the animal.

### Example 10: Detection of genes for PS/A production in isolates of S. aureus.

### Methods: DNA extraction from bacteria, PCR analysis and Souther blot analysis:

DNA preparation: Bacteria were inoculated in 10-2- ml of trypticase soy broth (TXB) and grown overnight at 37°C. The resulting growth was recovered by centrifugation. The bacterial cells were resuspended in lysis buffer (25mM Tris, pH 8.0, 25 mM EDTA, 300 mM sucrose). Cells were then incubated with lysostaphin (0.2 mg/ml) and lysozyme (2 mg/ml) for 1-3 hours at 37°C. Following this enzyme digestion, 0.5 ml of 5% sodium dodecyl sulfate (SDS) in 45% etanol was added, vortexed and incubated for 30 min at room temperature. One ml of buffered phenol/chloroform/isoamyl alcholo (volume ratios of 27:12:1) was then added followed by vortexing and centrifugation (15 min, 5000 rpm, 4°C). The upper aqueous layer was removed and added to chloroform:Isoamyl alchol (24:1 ratio). Again this was centrifuged (15 min, 5000 rpm, 4°C) the DNA was recovered in the upper aqueous layer, then precipitated by addition of 95% ethanol to amount equal to double the starting volume, and the precipitated DNA spooled onto a glass rod. The DNA was then dissolved in distilled water.

Restriction enzyme digestion of NDA for Southern blot analysis was done according to the manufacturer's protocol (Gibco BRL, Grand Island, NY). The DNA probe was labeled using the ECL protocol (Amersham International plc, Buckinghamshire, England) following the manufacturer's instructions.

PCR was performed using a touch down protocol. Briefly, primers (SEQ. ID. NO. 2-3) were added to the DNA obtained from the Staphylococcal strains (200nM final concentration) and the thermal cycler programmed to perform DNA melting at 95 °C and polymerase extensions starting at 60°C with decreasing annealing temperatures of 0.5°C used at each cycle until reaching 45°C.
Results: Using the polymerase chain reaction (PCR) we have shown that isolates of *S*. *aureus* have the genes to make PS/A. These genes are contained in a locus designated *ica* and were originally isolated and sequenced by Heilmann et al. (Heilmann, C., O. Schweitzer, C. Gerke, N. Vanittanakom, D. Mack and F. Gotz. 1996. Molecular basis of intercellular adhesion in the biofilm-forming Staphylococcus epidermidis. Molec. Microbiol. 20:1083*).* We demonstrate herein that the *ica* locus encodes proteins that synthesize the poly-N-succinyl glucosamine molecule PS/A and not the poly-N-acetylated polyglucosamine molecule termed PIA.

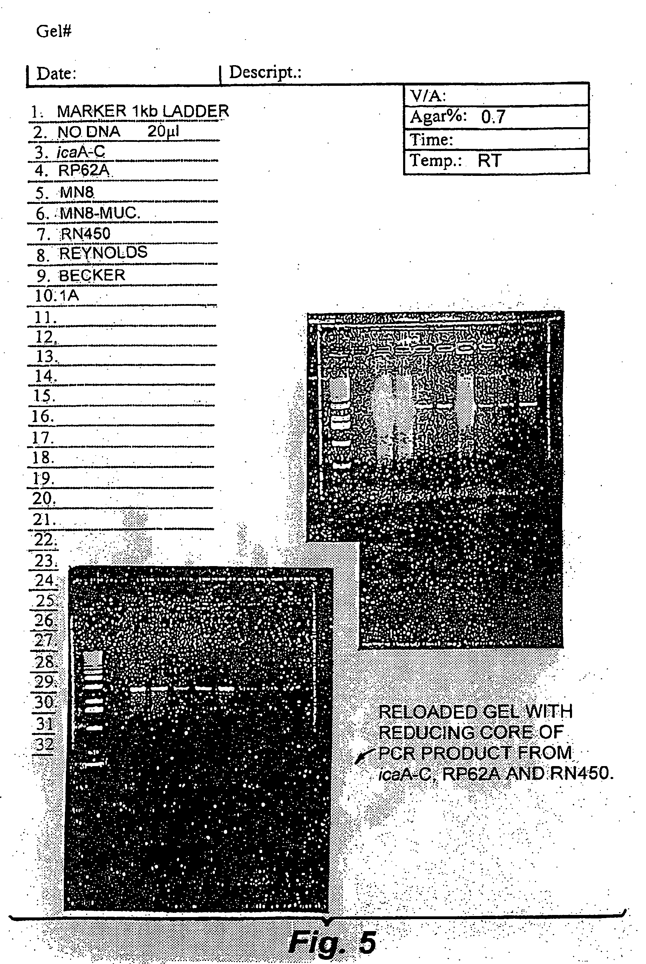

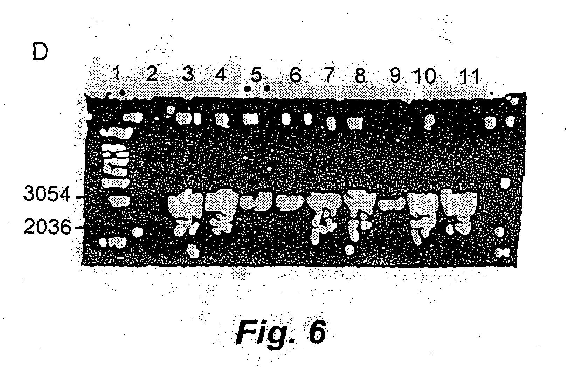

Primers were designed for the PCR analysis based on the sequence of the *ica* genes to amplify a fragment of DNA of 2.7 kB (SEQ ID No: 2 and 3). When these primers were mixed with DNA isolated from *S*. *aureus* strains MN8, MN8 mucoid, RN450, Becker, Reynolds and lA , along with DNA from *S*. *epidermidis* strain RP62A, from which the *ica* genes had originally been cloned, and used in a PCR reaction we found that all of the *S*. *aureus* strains had the *ica* locus (Figure 5). When the amplified 2.7 kB fragment was labeled and used as a probe in a Southern blot reaction of DNA extracted from these *S*. *aureus* strains, we also detected the presence of the genes in the *ica* locus (Figure 6). We have thus shown that the genes needed to make to proteins to synthesize the PS/A antigen are present in *S. aureus.*

The foregoing written specification is considered to be sufficient to enable one skilled in the art to practice the invention. The present invention is not to be limited in scope by examples provided, since the examples are intended as a single illustration of one aspect of the invention and other functionally equivalent embodiments are within the scope of the invention. Various modifications of the invention in addition to those shown and described herein will become apparent to those skilled in the art from the foregoing description and fall within the scope of the appended claims.

## Claims

1. A method of preparing a pure capsular polysaccharide/adhesin that is greater than 92% free of contaminants comprising:
(a) extracting a crude capsular polysaccharide/adhesin preparation from a coagulase-negative or coagulase-positive *Staphylococci* bacterial culture using low pH and heat,
(b) isolating a high-molecular weight capsular polysaccharide/adhesin-enriched material from the crude capsular polysaccharide/adhesin preparation,
(c) precipitating an impure capsular polysaccharide/adhesin from the high-molecular weight capsular polysaccharide/adhesin-enriched material,
(d) incubating the impure capsular polysaccharide/adhesin with a base to produce a semi-pure capsular polysaccharide/adhesin preparation,
(e) neutralizing the preparation with an acid, and
(f) incubating the neutralized preparation in hydrofluoric acid to produce the pure capsular polysaccharide/adhesin.

2. A method as claimed in claim 1, wherein the coagulase positive *Staphylococci* bacterial culture is a *Staphylococcus aureus* culture.

3. A method as claimed in any of claims 1 or 2 wherein the bacterial culture is a culture of a bacterial strain that expresses the *ica* locus.

4. A method as claimed in any of claims 1 to 3 wherein extracting the crude capsular polysaccharide/adhesin preparation from a bacterial culture in step (a) is at pH 5.0.

5. A method as, claimed in any of claims 1 to 4 wherein extracting the crude capsular polysaccharide/adhesin preparation from a bacterial culture in step (a) is at a temperature of 65°C.

6. A method as claimed in claim 5 wherein the temperature of 65°C is for 90 mins.

7. A method as claimed in any of claims 1 to 6 wherein extracting the crude capsular polysaccharide/adhesin preparation from a bacterial culture in step (a) is with the use of divalent cations.

8. A method as claimed in claim 7 wherein MgCl₂ is added to give a final concentration of 100mM.

9. A method as claimed in any of claims 1 to 8 wherein the high-molecular weight capsular polysaccharide/adhesin-enriched material from the crude capsular polysaccharide/adhesin preparation in (b) is isolated by molecular sieve chromatography.

10. A method as claimed in claim 9 wherein the high-molecular weight capsular polysaccharide/adhesin-enriched material elutes in void-volume fractions (>100,000 Da).

11. A method as claimed in any of claims 1 to 10, wherein step (c) is performed using 95% ethanol.

12. A method as claimed in any of claims 1 to 11 wherein the base in step (d) is NaOH.

13. A method as claimed in claim 12, wherein step (d) is performed by the addition of NaOH to a final concentration of 500 mM.

14. A method as claimed in claim 12 or 13, wherein the incubation of step (d) occurs at about 22°C for about 2 hours.

15. A method as claimed in any of claims 1 to 14, wherein the acid in step (e) is HCl.

16. A method as claimed in any of claims 1 to 15 wherein the neutralized preparation is incubated in 24% (v/v) hydrofluoric acid in step (f) to produce the pure capsular polysaccharide/adhesin.

17. A method as claimed in claim 16, wherein the incubation of step (f) occurs at about 4°C for about 48 hours.

18. A method as claimed in claim 9 further comprising enzymatic treatment.

19. A method as claimed in any preceding claim wherein the pure capsular polysaccharide/adhesin has a molecular weight of greater than 100,000 Daltons.

20. A polysaccharide comprising a pure capsular polysaccharide/adhesin that is greater than 92% free of contaminants prep arable according to the method of any of claims 1 to 19 which is formulated as a vaccine.

21. A polysaccharide as claimed in claim 20 and having a molecular weight of greater than 100,000 Daltons.

22. A polysaccharide as claimed in claim 20 or 21 for use in medicine.

23. A polysaccharide as claimed in claim 22, to treat infection by *Staphylococci* by inducing active immunity to *Staphylococci.*

24. A polysaccharide as claimed in claim 22 or claim 23, wherein immunity to infection by *Staphylococcus aureus* or *Staphylococcus epidermis* is induced.

25. A composition comprising a polysaccharide as claimed in any one of the preceding claims, and a carrier compound conjugated to the capsular polysaccharide/adhesin.

26. A composition as claimed in claim 25, wherein the carrier compound is conjugated to the capsular polysaccharide/adhesin through a linker.

27. Use of the pure capsular polysaccharide/adhesin as claimed in claim 20 or 21 optionally together with an adjuvant, in the preparation of a medicament for producing an antibody response.

28. A method for producing an antibody against the pure capsular polysaccharide/adhesin as claimed in claim 20 or 21 comprising immunizing a non-human animal with multiple subcutaneous, intraperitoneal or intradermal injection with the pure capsular polysacchaxide/adhesin as claimed in claim 20 or 21 in conjunction with an adjuvant.

29. A method as claimed in claim 28 wherein the antibody is a monoclonal antibody and the method further comprises immortalizing spleen cells isolated from the immunised animal.

## Patentansprüche

1. Verfahren zum Zubereiten eines reinen kapsulären Polysaccharides/Adhesins, das zu mehr als 92% frei von Verunreinigungen ist, umfassend:
(a) Extrahieren einer Rohzubereitung des kapsulären Polysaccharides/Adhesins aus einer Coagulase-negativen oder Coagulase-positiven bakteriellen Staphylokokken-Kultur unter Verwendung eines niedrigen pH-Wertes und von Hitze,
(b) Isolieren eines hinsichtlich des kapsulären Polysaccharides/Adhesins mit hohem Molekulargewicht angereicherten Materials aus der Rohzubereitung des kapsulären Polysaccharides/Adhesins,
(c) Präzipitieren eines unreinen kapsulären Polysaccharides/Adhesins aus dem hinsichtlich des kapsulären Polysaccharides/Adhesins mit hohem Molekulargewicht angereicherten Material,
(d) Inkubieren des unreinen kapsulären Polysaccharides/Adhesins mit einer Base, um eine halbreine Zubereitung des kapsulären Polysaccharides/Adhesins zu produzieren,
(e) Neutralisieren der Zubereitung mit einer Säure, und
(f) Inkubieren der neutralisierten Zubereitung in Flusssäure, um das reine kapsuläre Polysaccharid/Adhesin zu produzieren.

2. Verfahren wie in Anspruch 1 beansprucht, wobei die Coagulase-positive bakterielle Staphylokokken-Kultur eine *Staphylococcus aureus*-Kultur ist.

3. Verfahren wie in einem der Ansprüche 1 oder 2 beansprucht, wobei die bakterielle Kultur eine Kultur eines bakteriellen Stammes ist, der den *ica*-Locus exprimiert.

4. Verfahren wie in einem der Ansprüche 1 bis 3 beansprucht, wobei das Extrahieren der Rohzubereitung des kapsulären Polysaccharides/Adhesins aus einer bakteriellen Kultur in Schritt (a) bei pH 5,0 stattfindet.

5. Verfahren wie in einem der Ansprüche 1 bis 4 beansprucht, wobei das Extrahieren der Rohzubereitung des kapsulären Polysaccharides/Adhesins aus einer bakteriellen Kultur in Schritt (a) bei einer Temperatur von 65 °C stattfindet.

6. Verfahren wie in Anspruch 5 beansprucht, wobei die Temperatur von 65 °C 90 Min. lang anhält.

7. Verfahren wie in einem der Ansprüche 1 bis 6 beansprucht, wobei das Extrahieren der Rohzubereitung des kapsulären Polysaccharides/Adhesins aus einer bakteriellen Kultur in Schritt (a) unter der Verwendung von zweiwertigen Kationen stattfindet.

8. Verfahren wie in Anspruch 7 beansprucht, wobei MgCl₂ hinzugegeben wird, um eine Endkonzentration von 100 mM zu ergeben.

9. Verfahren wie in einem der Ansprüche 1 bis 8 beansprucht, wobei das hinsichtlich des kapsulären Polysaccharides/Adhesins mit hohem Molekulargewicht angereicherte Material aus der Rohzubereitung des kapsulären Polysaccharides/Adhesins in (b) durch Molekularsiebchromatographie isoliert wird.

10. Verfahren wie in Anspruch 9 beansprucht, wobei das hinsichtlich des kapsulären Polysaccharides/Adhesins mit hohem Molekulargewicht angereicherte Material in Hohlraumvolumenfraktionen (>100 000 Da) eluiert.

11. Verfahren wie in einem der Ansprüche 1 bis 10 beansprucht, wobei Schritt (c) unter Verwendung von 95%-igem Ethanol durchgeführt wird.

12. Verfahren wie in einem der Ansprüche 1 bis 11 beansprucht, wobei die Base in Schritt (d) NaOH ist.

13. Verfahren wie in Anspruch 12 beansprucht, wobei Schritt (d) durch die Zugabe von NaOH zu einer Endkonzentration von 500 mM durchgeführt wird.

14. Verfahren wie in Anspruch 12 oder 13 beansprucht, wobei die Inkubation von Schritt (d) etwa 2 Stunden lang bei etwa 22 °C stattfindet.

15. Verfahren wie in einem der Ansprüche 1 bis 14 beansprucht, wobei die Säure in Schritt (e) HCl ist.

16. Verfahren wie in einem der Ansprüche 1 bis 15 beansprucht, wobei die neutralisierte Zubereitung in Schritt (f) in 24 % (Vol/Vol) Flusssäure inkubiert wird, um das reine kapsuläre Polysaccharid/Adhesin zu produzieren.

17. Verfahren wie in Anspruch 16 beansprucht, wobei die Inkubation von Schritt (f) etwa 48 Stunden lang bei etwa 4 °C stattfindet.

18. Verfahren wie in Anspruch 9 beansprucht, weiter umfassend eine enzymatische Behandlung.

19. Verfahren wie in einem vorhergehenden Anspruch beansprucht, wobei das reine kapsuläre Polysaccharid/Adhesin ein Molekulargewicht von mehr als 100 000 Dalton aufweist.

20. Polysaccharid umfassend ein reines kapsuläres Polysaccharid/Adhesin, das zu mehr als 92% frei von Verunreinigungen ist, herstellbar gemäß dem Verfahren nach einem der Ansprüche 1 bis 19, das als Impfstoff formuliert ist.

21. Polysaccharid wie in Anspruch 20 beansprucht mit einem Molekulargewicht von mehr als 100 000 Dalton.

22. Polysaccharid wie in Anspruch 20 oder Anspruch 21 beansprucht zur Verwendung in der Medizin.

23. Polysaccharid wie in Anspruch 22 beansprucht, um eine Infektion durch Staphylokokken durch das Induzieren einer aktiven Immunität gegen Staphylokokken zu behandeln.

24. Polysaccharid wie in Anspruch 22 oder Anspruch 23 beansprucht, wobei eine Immunität gegen eine Infektion durch *Staphylococcus aureus* oder *Staphylococcus epidermis* induziert wird.

25. Zusammensetzung umfassend ein Polysaccharid wie in einem der vorhergehenden Ansprüche beansprucht, und eine Trägerverbindung, die mit dem kapsulären Polysaccharid/Adhesin konjugiert ist.

26. Zusammensetzung wie in Anspruch 25 beansprucht, wobei die Trägerverbindung über einen Linker mit dem kapsulären Polysaccharid/Adhesin konjugiert ist.

27. Verwendung des reinen kapsulären Polysaccharides/Adhesins wie in Anspruch 20 oder 21 beansprucht, optional zusammen mit einem Adjuvans, zur Herstellung eines Medikamentes zum Hervorrufen einer Antikörperantwort.

28. Verfahren zum Herstellen eines Antikörpers gegen das wie in Anspruch 20 oder 21 beanspruchte reine kapsuläre Polysaccharid/Adhesin, umfassend das Immunisieren eines nichtmenschlichen Tieres mit einer mehrfachen subkutanen, intraperitonealen oder intradermalen Injektion mit dem wie in Anspruch 20 oder 21 beanspruchten reinen kapsulären Polysaccharid/Adhesin zusammen mit einem Adjuvans.

29. Verfahren wie in Anspruch 28 beansprucht, wobei der Antikörper ein monoklonaler Antikörper ist und das Verfahren weiterhin das Immortalisieren von aus dem immunisierten Tier isolierten Milzzellen umfasst.

## Revendications

1. Procédé pour préparer un polysaccharide capsulaire / une adhésine pur(e) qui est exempt(e) de contaminants à plus de 92 %, consistant à :
(a) extraire une préparation de polysaccharide capsulaire / adhésine brute à partir d'une culture bactérienne de *Staphylocoques* à coagulase négative ou à coagulase positive en utilisant un faible pH et la chaleur,
(b) isoler un matériau enrichi en polysaccharide capsulaire adhésine de haut poids moléculaire à partir de la préparation de polysaccharide capsulaire / adhésine brute ;
(c) précipiter un polysaccharide capsulaire / une adhésine impur(e) à partir du matériau enrichi en polysaccharide capsulaire / adhésine de haut poids moléculaire,
(d) incuber le polysaccharide capsulaire / l'adhésine impur(e) avec une base pour produire une préparation de polysaccharide capsulaire / adhésine semi-pure,
(e) neutraliser la préparation avec un acide, et
(f) incuber la préparation neutralisée dans de l'acide fluorhydrique afin de produire le polysaccharide capsulaire / l'adhésine pur(e).

2. Procédé selon la revendication 1, où la culture bactérienne de *Staphylocoques à* coagulase négative est une culture de *Staphylococcus aureus.*

3. Procédé selon l'une quelconque des revendications 1 ou 2, où la culture bactérienne est une culture d'une souche bactérienne qui exprime le locus *ica.*

4. Procédé selon l'une quelconque des revendications 1 à 3, où l'extraction de la préparation de polysaccharide capsulaire / adhésine brute à partir d'une culture bactérienne à l'étape (a) est réalisée à un pH de 5,0.

5. Procédé selon l'une quelconque des revendications 1 à 4, où l'extraction de la préparation de polysaccharide capsulaire / adhésine brute à partir d'une culture bactérienne à l'étape (a) est réalisée à une température de 65 °C.

6. Procédé selon la revendication 5, où la température de 65 °C est appliquée pendant 90 minutes.

7. Procédé selon l'une quelconque des revendications 1 à 6, où l'extraction de la préparation de polysaccharide capsulaire / adhésine brute à partir d'une culture bactérienne à l'étape (a) est réalisée en utilisant des cations divalents.

8. Procédé selon la revendication 7, où du MgCl₂ est ajouté pour obtenir une concentration finale de 100 mM.

9. Procédé selon l'une quelconque des revendications 1 à 8, où le matériau enrichi en polysaccharide capsulaire / adhésine issu de la préparation de polysaccharide capsulaire/adhésine brute en (b) est isolé par une chromatographie de tamisage moléculaire.

10. Procédé selon la revendication 9, où le matériau enrichi en polysaccharide capsulaire / adhésine de haut poids moléculaire est élué dans des fractions de volume mort (> 100000 Da).

11. Procédé selon l'une quelconque des revendications 1 à 10, où l'étape (c) est réalisée en utilisant de l'éthanol à 95 %.

12. Procédé selon l'une quelconque des revendications 1 à 11, où la base à l'étape (d) est du NaOH.

13. Procédé selon la revendication 12, où l'étape (d) est réalisée en ajoutant du NaOH à une concentration finale de 500 mM.

14. Procédé selon l'une quelconque des revendications 12 ou 13, où l'incubation de l'étape (d) est réalisée à environ 22 °C pendant environ 2 heures.

15. Procédé selon l'une quelconque des revendications 1 à 14, où l'acide à l'étape (e) est du HCl.

16. Procédé selon l'une quelconque des revendications 1 à 15, où la préparation neutralisée est incubée dans de l'acide fluorhydrique à 24 % (v/v) à l'étape (f) afin de produire le polysaccharide capsulaire / l'adhésine pur(e).

17. Procédé selon la revendication 16, où l'incubation de l'étape (f) est réalisée à environ 4 °C pendant environ 48 heures.

18. Procédé selon la revendication 9, comprenant en outre un traitement enzymatique.

19. Procédé selon l'une quelconque des revendications précédentes, où le polysaccharide capsulaire / l'adhésine pur(e) possède un poids moléculaire supérieur à 100000 Daltons.

20. Polysaccharide comprenant un polysaccharide capsulaire / une adhésine pur(e) qui est exempt(e) de contaminants à plus de 92 % pouvant être préparé selon le procédé de l'une quelconque des revendications 1 à 19, qui est formulé en tant que vaccin.

21. Polysaccharide selon la revendication 20 et ayant un poids moléculaire supérieur à 100000 Daltons.

22. Polysaccharide selon la revendication 20 ou 21, destiné à une utilisation en médecine.

23. Polysaccharide selon la revendication 22, pour traiter une infection par des *Staphylocoques* en induisant une immunité active contre des *Staphylocoques.*

24. Polysaccharide selon la revendication 22 ou la revendication 23, où une immunité contre une infection par *Staphylococcus aureus* ou *Staphylococcus epidermis* est induite.

25. Composition comprenant un polysaccharide selon l'une quelconque des revendications précédentes, et un composé véhicule conjugué au polysaccharide capsulaire / à l'adhésine.

26. Composition selon la revendication 25, où le composé véhicule est conjugué au polysaccharide capsulaire / à l'adhésine par l'intermédiaire d'un segment de liaison.

27. Utilisation du polysaccharide capsulaire / de l'adhésine pur(e) selon la revendication 20 ou 21, facultativement avec un adjuvant, dans la préparation d'un médicament pour produire une réponse d'anticorps.

28. Procédé pour produire un anticorps contre le polysaccharide / l'adhésine pur(e) selon la revendication 20 ou 21, consistant à immuniser un animal non humain par de multiples injections sous-cutanées, intrapéritonéales ou intradermiques, avec le polysaccharide capsulaire / l'adhésine pur(e) selon la revendication 20 ou 21 en conjonction avec un adjuvant.

29. Procédé selon la revendication 28, où l'anticorps est un anticorps monoclonal et le procédé consiste en outre à immortaliser des cellules spléniques isolées à partir de l'animal immunisé.


### SEQUENCE LISTING

<110> The Brigham and Women's Hospital, Inc.
   Pier, Gerald B.
   McKenney, David
   Wang, Ying
<120> CAPSULAR POLYSACCHARIDE ADHESIN ANTIGEN
   PREPARATION, PURIFICATION AND USE
<130> B0801/7144/HCL
<150> US 60/093,117
   <151> 1998-07-15
<160> 3
<170> FastSEQ for Windows Version 3.0
<210> 1
   <211> 4500
   <212> DNA
   <213> Staphylococcus Epidermidis
<400> 1
   tttgaaatct cgaatttgtt acatactagt tacaaaaatt atttttttaa aaatacattt     60
   aacagtgaat atacttggtc tttaaaacgg tttttactgt cctcaataat cccgaatttt    120
   tgtgaaaagg aggctctaaa ataccaagtc tcaagaaaaa gaagaattaa gtttataaag    180
   tcctctttat ccaaagcgat gtgcgtagga tcataatact ttatcaattc atcatgtaag    240
   gtagtattaa tttcttgaag atggtgtttg atttctgaat tcagtgcttc tggagcacta    300
   gataattgaa catataattt aatatatctc tcatcaacgt cgaatataaa tttgaataaa    360
   aactggtaaa gtccgtcaat ggaataatta tcatcatggt tcctaagcaa aaaatctata    420
   aagtaattga aacaattctc aacacttttt cgatatattt cttccttatt atcgtaatga    480
   taatatagac tagccttttt tatatttaca cttttagaaa tatcatcaag tgtagtacca    540
   tcgtacccct tttcggaaaa taaggttatt gcgttatcaa taatcttatc tttcaattct    600
   aaaatctccc ccttattcaa ttttctaaaa atatattaca gaaaaattaa gttaaaatta    660
   caaatattac tgtttcagta taacaacatt ctattgcaaa ttgaaatact ttcgattagc    720
   atatgcttta caacctaact aacgaaaggt aggtgaaaaa atgcatgtat ttaacttttt    780
   acttttctat ccaattttta tgtcaattta ctggatagta ggatcgattt actatttttt    840
   tattaaagaa aaacccttta atcgatcatt gttagtaaaa tctgaacatc aacaagttga    900
   aggcatctcc tttttattag cttgctacaa tgaaagtgaa acagttcaag acacgctttc    960
   tagtgtttta tctctagaat atcctgaaaa agaaattatc attatcaatg atggaagttc   1020
   tgataatact gctgaaatca tctatgactt caagaaaaat catgatttta aatttgttga   1080
   cctcgaagtc aatagaggta aagctaatgc actcaatgag ggaatcaaac aagcatctta   1140
   cgaatatgtt atgtgtttag atgctgacac tgtcattgat gacgatgcgc ctttttatat    1200
   gattgaagac tttaaaaaga atccaaaatt aggcgcagtt acaggtaatc cacgtattcg   1260
   taataaaagt tctattttag gaaaaataca gaccattgaa tatgcaagta ttattggttg   1320
   tatcaagcga agtcaatctc ttgcaggagc aatcaatact atttcaggtg ttttcacact   1380
   atttaaaaaa agtgcactca aagatgtagg ttattgggat actgacatga ttactgagga   1440
   tattgctgtt tcatggaaac tccatctttt tgattacgaa attaagtacg aaccacgtgc   1500
   tctatgctgg atgttagtgc ctgaaactat aggtggttta tggaaacaaa gggttcgatg   1560
   ggctcaaggc gggcatgaag tacttttaag agacttttgg ccaacaatta aaactaagaa   1620
   attatcacta tatattttaa tgtttgaaca aatcgcatcg attacatggg tctacatcgt   1680
   actatgttat ttatcttttt tagtaatcac agccaacatc ttagattaca catatttaaa    1740
   atatagtttt tcaatctttt tcttttcatc ctttacgatg acctttatca atatcatcca    1800
   atttacagtt gccttattta ttgacagtcg ctacgaaaag aaaaatatag ttggcctgat    1860
   atttttaagt tggtatccaa cgttatactg ggttatcaat gccgcagttg tcattatggc    1920
   atttcctaaa gcattaaaaa gaaagaaagg tggctatgct acatggtcaa gcccagacag    1980
   aggcaatatc caacggtaac ctcttattta aatatagtta gggagagctt atttattact    2040
   atatccggag tattttggat gtattgtatc gttgtgatga ttgtttatat aggaactctt    2100
   atcaattctc aaatggaaag tgttataaca atacgtattg cattaaatgt tgaaaacacg    2160
   gaaatttaca aattattcgg atggatgagt ttgtttgtac ttattatatt tatctttttt    2220
   acatttagtc tcgcgtttca aaaatataag aaaggtcgtg acatatgaaa cctttcaaat    2280
   taatctttat tagcgcattg atgatattaa taatgacgaa tgcaacacca atatcacacc    2340
   tgaatgctca agctaatgaa gaaaacaaga agttaaagta cgaaaaaaat agcgcactcg    2400
   cgttaaacta tcacagagta agaaaaaagg atcctttgaa tgactttata tcattactat    2460
   ctgggagtaa ggaaattaaa aattatagtg tcactgatca agaatttaaa tcacaaattc    2520
   aatggcttaa agcacacgac gcaaagtttt taactttgaa agaatttatt aaatataaag    2580
   aaaaaggtaa atttcctaaa agaagtgttt ggattaactt tgatgatatg gatcaaacga    2640
   tttatgacaa tgcctttcct gttttgaaaa aatatcatat tccagcaaca ggttttctta    2700
   ttacgaacca cattggttct accaattttc ataatttaaa tttactttca aaaaagcaat    2760
   tagatgaaat gtatgaaaca ggcttatggg actttgaatc tcatactcat gatttacacg    2820
   ctcttaagaa aggcaataaa tcgaagtttt tagattcgtc tcaatctgtt gctagtaaag    2880
   atattaaaaa aagcgaacac tatttaaata aaaactaccc aaaaaatgaa cgcgcacttg    2940
   cttacccata cggattaatt aatgacgaca aaataaaagc tatgaaaaaa aatggaattc    3000
   aatatgggtt tacacttcag gaaaaagctg tcacaccaga tgccgataac tatagaattc    3060
   cacgtatttt agtaagtaat gatgcatttg aaacgctaat aaaggaatgg gacggattcg    3120
   atgaagaaaa ataaacttga attagtgtat ttacgtgcgt ttatttgtgt cataatcatc    3180
   gtgacacact tactaacgca aatcacttta gaaaatgaac agatgtctga tagttcactc    3240
   atattgcaat attatatacg caatattttt attttcggca cccctagttt tataatattg    3300
   tctcaattat taacaacatt aaattacgaa tcagtaacta taaattatct tttttcaaga    3360
   tttaagtata tttttattcc atatctttta atcggcttgt tctatagtta tagtgaatca    3420
   cttatcaccg cttcttcttt taaaaagcag tttatcgaaa atgttgtttt aggacaatgg    3480
   tatggctatt tcattatcat aattatgcag ttctttgttc tatcttatat catttacaaa    3540
   attaatttta gattgttcaa tagtaaaatt ttgctgcttt tagcatttat agtccaacaa    3600
   tcttatctac attatttttt gaataatgac acttttcatc aattcatgac tcattattat    3660
   ccattaagcg aaaatacaat gatattagga tggatattct actttttctt aggtggttac    3720
   attggctata attatgaaaa aatattatct ttcttagaaa aatatttaat tatagttatt    3780
   atgttaactt taggcgcata tgttttattt atcgctgttt ccggtagtga ttattggaat    3840
   gtcacaagct ttacttatac gttaacatta tataatagtg tcatgttctt cttattacta    3900
   ggagtctgta tgcactttaa aactatgtta ttaaatacta ttaaagctat tagtgcattt    3960
   tcctttttca tttatttgtt gcacccaatt atcttagatt ctctttttgc ttacaccaac    4020
   atatttgaag ataatacaat tgttttcttg gcgatttcac ttttaatgat tctaggaatt    4080
   tgtataggcg tcggaatgat gttaagagag ttttatatat tcagatttgt aattggaaaa    4140
   caaccgtaca agttacaatt tgaccaatac cagcctaact ggaattaata aaaaaagtcc    4200
   cttattcaag ctatggctta tgtatgtgct tgaataaggg attttatctt actatagttt    4260
   cacattatga aaataaattt tttaatattc tgtataaaga gcctaataat tgaaagaaat    4320
   cacctgtcat gtatctcact cctatctata taagattacg ttaggtttat accctatatc    4380
   attttattta tttgttgatg ttaattgttc actttgtact aaatcatcag caagaccatg    4440
   ccaaaactgt tgcaattcat ctctagtacg ttttgtatct gtactgtctt gtcctacaaa    4500
<210> 2
   <211> 20
   <212> DNA
   <213> Artificial Sequence
<220>
<223> Primer
<400> 2
   tgcactcaat gagggaatca 20
<210> 3
   <211> 21
   <212> DNA
   <213> Artificial Sequence
<220>
   <223> Primer
<400> 3
   aatcactacc ggaaaacagc g 21